# FASCICULE DE BREVET EUROPEEN

(11) **EP 0 984 925 B1**
(45) Date de publication et mention de la délivrance du brevet: **08.12.2004**
(21) Numéro de dépôt: 98928356.9
(22) Date de dépôt: 25.05.1998
(51) Int. Cl.: C07C 279/12, C07C 257/14, C12N 15/87

(54) **COMPOSES, LEUR PREPARATION ET LEUR UTILISATION POUR LE TRANSFERT D'ACIDES NUCLEIQUES DANS LES CELLULES**
VERBINDUNGEN, IHRE HERSTELLUNG UND IHRE VERWENDUNG FÜR DEN TRANSFER VON NUCLEINSÄUREN IN ZELLEN
COMPOUNDS, PREPARATION AND USE FOR TRANSFERRING NUCLEIC ACIDS INTO CELLS

(30) Priorité: 28.05.1997 FR 9706549
(43) Date de publication de la demande: 15.03.2000
(73) Titulaire: Aventis Pharma S.A., 92165 Antony Cédex (FR)
(72) Inventeur: BYK, Gérardo, F-94000 Créteil (FR); DUBERTRET, Catherine, F-92310 Sèvres (FR); SCHERMAN, Daniel, F-75012 Paris (FR)
(74) Mandataire: Peaucelle, Chantal
(86) Numéro de dépôt international: PCT/FR1998/001041
(87) Numéro de publication internationale: WO 1998/054130

(56) Documents cités:
- WO-A-95/14381
- WO-A-96/01840
- WO-A-96/01841
- WO-A-96/17823
- WO-A-96/25508
- WO-A-97/18185

## Description

La présente invention concerne de nouveaux composés de transfert d'acides nucléiques dans les cellules. Ces nouveaux composés sont plus particulièrement apparentés à la famille des lipopolyamines, et comportent des fonctions amidines. Ces composés sont utilisables pour transférer des acides nucléiques d'intérêt dans différents types cellulaires, aussi bien *in vitro* que *in vivo* ou *ex vivo*.

Avec le développement des biotechnologies, la possibilité de transférer efficacement des acides nucléiques dans les cellules est devenue une nécessité. Il peut s'agir du transfert d'acides nucléiques dans des cellules *in vitro*, par exemple pour la production de protéines recombinantes, ou au laboratoire, pour l'étude de la régulation de l'expression de gènes, le clonage de gènes, ou toute autre manipulation impliquant l'ADN. Il peut également s'agir du transfert d'acides nucléiques dans des cellules in *vivo*, par exemple pour la création d'animaux transgéniques, la réalisation de vaccins, des études de marquage ou également des approches thérapeutiques. Il peut encore s'agir du transfert d'acides nucléiques dans des cellules *ex vivo*, dans des approches de greffes de moelle osseuse, d'immunothérapie ou d'autres méthodes impliquant le transfert de gènes dans des cellules prélevées d'un organisme, en vue de leur réadministration ultérieure.

Différents types de vecteurs synthétiques ont été développés pour améliorer le transfert des acides nucléiques dans les cellules. Parmi ces vecteurs, les lipides cationiques possèdent des propriétés intéressantes. Ces vecteurs sont constitués d'une partie polaire, cationique, interagissant avec les acides nucléiques, et d'une partie lipidique, hydrophobe, favorisant la pénétration cellulaire. Des exemples particuliers de lipides cationiques sont notamment les lipides monocationiques (DOTMA : Lipofectin®), certains détergents cationiques (DDAB), les lipopolyamines et en particulier la dioctadécylamidoglycyl spermine (DOGS) ou la 5-carboxyspermylamide de la palmitoylphosphatidylethanolamine (DPPES) dont la préparation a été décrite par exemple dans la demande de brevet EP 394 111. Une autre famille de lipopolyamines est représentée par les composés décrits dans la demande de brevet WO 97/18185, incorporée à la présente par référence.

La présente invention concerne un nouveau type d'agents de transfert d'acides nucléiques, possédant des propriétés particulièrement intéressantes. Plus précisément, les composés selon la présente invention sont des lipides cationiques portant une région cationique originale, conférant aux molécules des propriétés améliorées. Cette partie cationique est réprésentée plus précisément par une polyamine particulière, portant une ou plusieurs fonctions amidine.

Un premier objet de l'invention concerne plus précisément des composés sous forme D, L ou DL, ainsi que ses sels, de formule générale (I) : 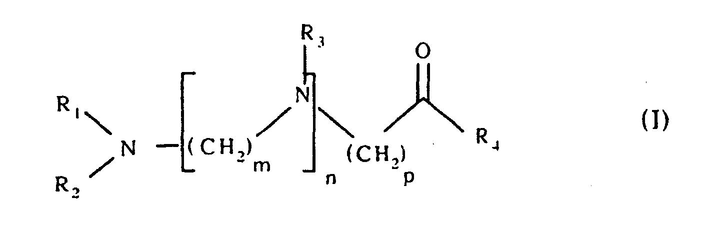 dans laquelle :
- R₁, R₂ et R₃ représentent indépendamment l'un de l'autre un atome d'hydrogène ou un groupement -(CH₂)_{q}-NRR' avec
   - q étant un entier de 1 à 6 inclus, les valeurs de q étant indépendantes les une des autres entre les différents groupements R₁, R₂ et R₃, et
   - R et R' représentent indépendamment l'un de l'autre un atome d'hydrogène ou un groupement de formule (II) : 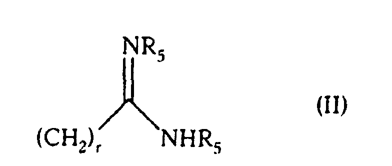
dans laquelle r est un nombre entier pouvant varier de 0 à 6 inclus, et les groupements R₅ représentent, indépendamment l'un de l'autre, un atome d'hydrogène ou un reste hydrocarboné,
étant entendu que l'un au moins des groupes R₁, R₂ et R₃ comporte au moins un groupement de formule (II),
- m et n représentent, indépendamment l'un de l'autre, un nombre entier pouvant varier de 1 à 6 inclus avec, lorsque n est supérieur à 1, m pouvant prendre des valeurs différentes et R₃ des significations différentes au sein de la formule générale (I),
- p représente un nombre entier pouvant varier de 1 à 6 inclus, et,
- R₄ représente un groupement de formule générale (III) : 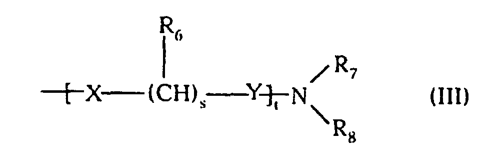
dans laquelle:
- R₇ et R₈ représentent indépendamment l'un de l'autre un atome d'hydrogène ou un groupement lipophile, l'un au moins des groupes R₇ et R₈ étant différent de l'hydrogène,
- t est un nombre entier choisi de 0 à 10 inclus avec R₆, X, Y et s pouvant avoir des significations différentes au sein des différents motifs [X-(CHR₆)_{S}-Y] lorsque t est un entier supérieur à 1,
- X représente un atome d'oxygène ou de soufre, ou un groupement amino ou alkylamino, le substituant alkyle étant linéaire ou ramifié et contenant 1 à 8 atomes de carbone,
- Y représente un groupement carbonyle ou un groupement méthylène,
- R₆ représente un atome d'hydrogène ou une chaîne latérale d'acide aminé naturel, le cas échéant substituée, et,
- s représente un entier variant entre 1 et 10 inclus avec lorsque s est égal à 1, R₆ représentant une chaine latérale d'acide aminé naturel, le cas échéant substituée, et lorsque s est supérieur à 1, R₆ représentant un atome d'hydrogène.

Le terme "forme DL" tel qu'utilisé précédemment signifie un mélange des formes D et L dans toute proportion, et par exemple dans des proportions égales.

On entend au sens de l'invention par "reste hydrocarboné" tout substituant alkyle, carbamate ou acyle aliphatique ou aromatique, éventuellement halogéné. Parmi les restes aliphatiques, on peut mentionner plus particulièrement les restes aliphatiques saturés ou insaturés, linéaires ou ramifiés, cycliques ou non, et éventuellement halogénés. Plus préférentiellement, il s'agit d'un reste aliphatique contenant 1 à 10 atomes de carbone. Notamment, il s'agit de substituants alcanoyle, alkyle et alkylcarbamate, tel que par exemple les substituants formyle, butyle, tert-butyle ou tert-butyl-carbamate. Selon une variante de l'invention, R₅ représente le tert-butylcarbamate.

Parmi les restes aromatiques, on peut citer plus particulièrement le benzyle et ses dérivés tels que le chlorobenzyle, le benzylcarbamate ou le chlorobenzylcarbamate.

De façon préférée, R₅ représente le tert-butylcarbamate, le benzylcarbamate ou le chlorobenzylcarbamate.

Dans une première variante de l'invention, l'un des groupes R₅ représente un atome d'hydrogène et l'autre un reste aliphatique contenant 1 à 10 atomes de carbone.

Dans une seconde variante de l'invention, l'un des groupes R₅ représente un atome d'hydrogène et l'autre un reste aromatique choisi de préférence parmi le benzyle et ses dérivés.

Dans une autre variante de l'invention, les deux groupes R₅ représentent des atomes d'hydrogène.

Avantageusement, lorsque R₁, R₂, et/ou R₃ sont différents de l'hydrogène et comprennent la formule générale (II), q prend la valeur 2 ou 3 et r vaut 0.

Préférentiellement, dans la formule générale (I), m est choisi parmi 2, 3 et 4.

Comme indiqué ci-avant, dans la formule (III), l'un au moins des groupes R₇ et R₈ représente un groupement lipophile. On entend au sens de l'invention par "groupement lipophile" tout groupement de type lipidique, hydrophobe, favorisant la pénétration cellulaire connu de l'homme du métier. Il peut s'agir en particulier d'une ou plusieurs chaînes aliphatiques grasses, d'un dérivé de stéroïde, d'un lipide naturel ou synthétique, de préférence capable de former des phases lamellaires ou héxagonales, ou éventuellement d'une combinaison de ceux-ci. Il peut s'agir plus particulièrement d'un radical aliphatique contenant 5 à 22 atomes de carbone, saturé ou non, linéaire ou ramifié, éventuellement halogéné. Il peut encore s'agir d'un dérivé de stéroïde, ou d'un groupement (CH₂)ᵤ-NH-R₉ dans lequel u est un nombre entier compris entre 2 et 6 inclus et R₉ est un radical acyle tel que par exemple le cholestérylformiate, l'arachidonyle ou l'acide cholique.

D'autres exemples de dérivés de stéroïde sont notamment le cholestérol, le cholestanol, le 3-α-5-cyclo-5-α-cholestan-6-β-ol, l'acide cholique, le cholestéryl-formiate, le chotestanylformiate, le 3α,5-cyclo-5α-cholestan-6β-yl formiate, la cholestérylamine, la 6-(1,5-diméthylhexyl)-3a,5a-diméthylhexadécahydrocyclopenta[a]cyclopropa[2,3]cyclopenta[1,2-f]naphta-lèn-10-ylamine, ou la cholestanylamine.

De manière préférentielle, le groupement lipophile est un radical aliphatique contenant entre 10 et 22 atomes de carbone, de préférence 14, 16, 17, 18 ou 19 atomes de carbone. On peut citer notamment les groupements lipophiles (CH₂)₁₃CH₃, (CH₂)₁₅CH₃, (CH₂)₁₆CH₃, (CH₂)₁₇CH₃, (CH₂)₁₈CH₃, et oléyle.

Dans un mode particulier de mise en oeuvre, les deux groupes R₇ et R₈ représentent des groupement lipophiles tels que définis ci-dessus. En particulier, dans un mode de réalisation préféré, chacun des groupes R₇ et R₈ représente une chaîne aliphatique contenant entre 5 et 22 atomes de carbone, et encore plus préférentiellement entre 12 et 22 atomes de carbone.

Selon une variante de l'invention, R₆ représente une chaîne latérale d'acide aminé naturel. Notamment, la chaîne latérale d'acide aminé naturel peut contenir des motifs amidinium comme par exemple la chaîne latérale de l'arginine. Cette chaîne latérale R₆ peut également , comme cela a été énoncé précédemment, être substituée par des groupements aliphatiques saturés ou insaturés, linéaires, ramifiés ou cycliques, et contenant entre 1 et 24 atomes de carbone. On peut citer à titre d'exemple les chaînes latérales d'acide aminé substituées par des radicaux cholestéryle, arachidonyle ou rétinoyle, des groupements mono- ou poly-aromatiques tels par exemple des dérivés benzyloxycarbonyle, benzylester, rhodaminyle, ou encore biotinyle substitués ou non.

Dans un mode de réalisation particulièrement avantageux, les composés revendiqués comprennent en outre un élément de ciblage permettant d'orienter le transfert de l'acide nucléique auquel ils sont associés. Cet élément de ciblage est de préférence incorporé, sur le composé de formule générale (I), au niveau de la chaîne latérale d'acide aminé figurée par le substituant R₆. Plus préférentiellement, l'élément de ciblage est lié, de manière covalente ou non covalente, au composé selon l'invention.

Il peut s'agir d'un élément de ciblage extracellulaire, permettant d'orienter le transfert de l'acide nucléique vers certains types cellulaires ou certains tissus souhaités (cellules tumorales, cellules hépatiques, cellules hématopoiétiques, etc...). A ce titre, il peut s'agir d'un ligand de récepteur cellulaire présent à la surface du type cellulaire ciblé comme par exemple un sucre, un folate, une transférrine, une insuline, une protéine asialo-orosomucoïde ou toute molécule bioactive reconnue par des récepteurs extracellulaires. Il peut également s'agir d'un élément de ciblage intracellulaire, permettant d'orienter le transfert de l'acide nucléique vers certains compartiments cellulaires privilégiés (mitochondries, noyau, etc) comme par exemple une séquence signal de localisation nucléaire (nls) qui privilégie l'accumulation de l'ADN transfecté au sein du noyau.

Plus généralement, les éléments de ciblage susceptibles d'être mis en oeuvre dans le cadre de l'invention incluent les sucres, les peptides, les oligonucléotides, les stéroïdes ou les lipides. Préférentiellement, il s'agit de sucres et/ou peptides tels que des anticorps ou fragments d'anticorps, des ligands de récepteurs cellulaires ou des fragments de ceux-ci, des récepteurs ou fragments de récepteurs, etc. En particulier, il peut s'agir de ligands de récepteurs de facteurs de croissance, de récepteurs de cytokines, de récepteurs de lectines cellulaires ou de récepteurs de protéines d'adhésion telles que les intégrines. On peut également citer le récepteur de la transferrine, des lipides HDL et des LDL. L'élément de ciblage peut également être un sucre permettant de cibler des lectines tels que les récepteurs asialoglycoprotéiques, ou encore un fragment Fab d'anticorps permettant de cibler le récepteur du fragment Fc des immunoglobulines.

De la même façon, il est possible d'envisager l'association à un composé de formule générale (I) d'un agent marqueur de type biotine, rhodamine, folate par exemple sur la chaine latérale d'acide aminé R₆. Cet agent marqueur peut également être une séquence peptidique ou pseudopeptidique linéaire ou cyclique comportant l'épitope Arg-Gly-Asp de reconnaissance des récepteurs primaires et/ou secondaires des protéines d'adhésion du type intégrines.

Une famille particulière de composés selon l'invention est celle pour laquelle R₁ comporte un groupement de formule (II), et R₂ et R₃ sont des atomes d'hydrogène.

Une seconde famille particulière de composés selon l'invention est celle pour laquelle R₁ et R₂ comportent chacun un groupement de formule (II), et R₃ est un atome d'hydrogène.

Une autre famille particulière de composés selon l'invention est celle pour laquelle R₁ et R₃ comportent chacun un groupement de formule (II), et R₂ est un atome d'hydrogène.

Une autre famille particulière de composés selon l'invention est celle dans laquelle R₁, R₂ et R₃ comportent chacun un groupement de formule (II).

Les nouveaux composés de formule générale (I) selon l'invention peuvent se présenter sous forme de sels, de préférence non toxiques et pharmaceutiquement acceptables. Ces sels non toxiques comprennent les sels formés avec des acides minéraux (acides chlorhydrique, sulfurique, bromhydrique, phosphorique, nitrique), formés avec des acides organiques (acides acétique, propionique, succinique, maléique, hydroxymaléique. benzoïque, fumarique, méthanesulfonique ou oxalique), ou encore formés avec des bases minérales (soude, potasse, lithine, chaux) ou organiques (amines tertiaires comme la triéthylamine, la pipéridine, la benzylamine).

A titre représentatif, on peut plus particulièrement citer les composés selon l'invention définis par les sous-formules générales suivantes : 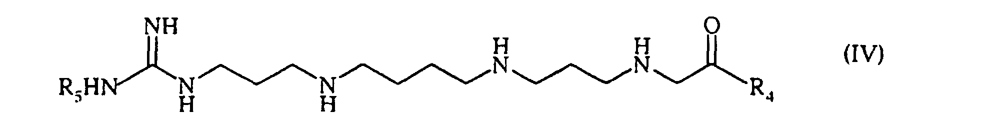 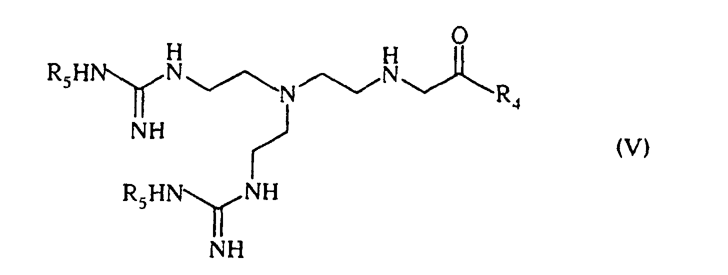 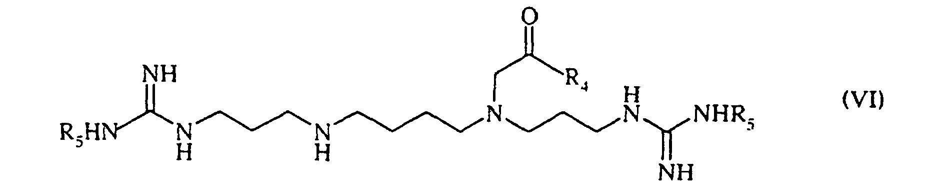 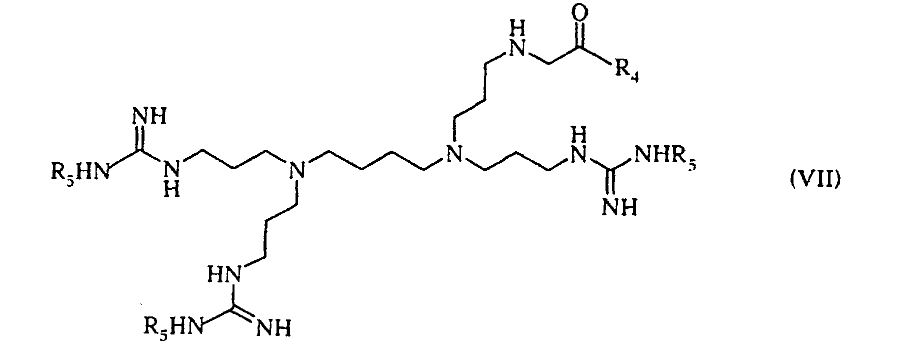 R₄ et R₅ étant définis comme précédemment.

A titre illustratif d'agents de transfert d'acides nucléiques selon la présente invention, on peut citer les composés de formules suivantes :

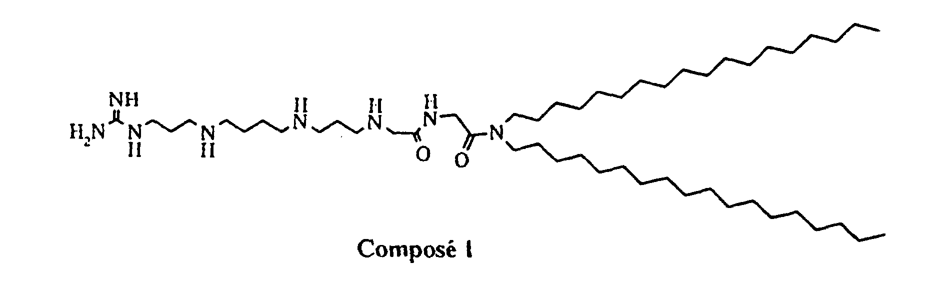

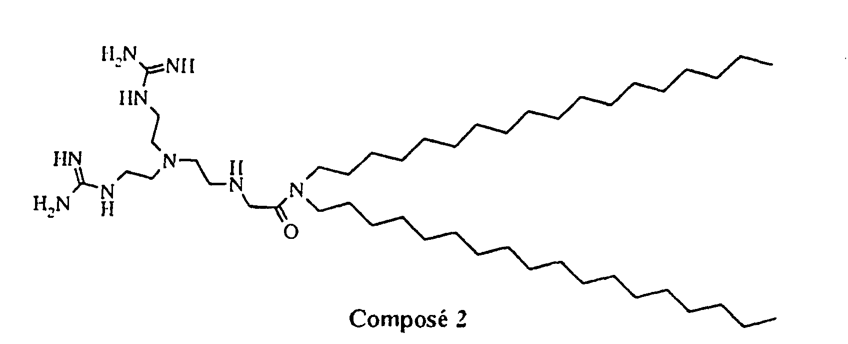

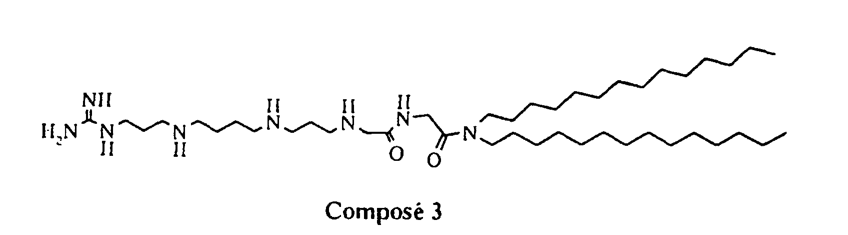

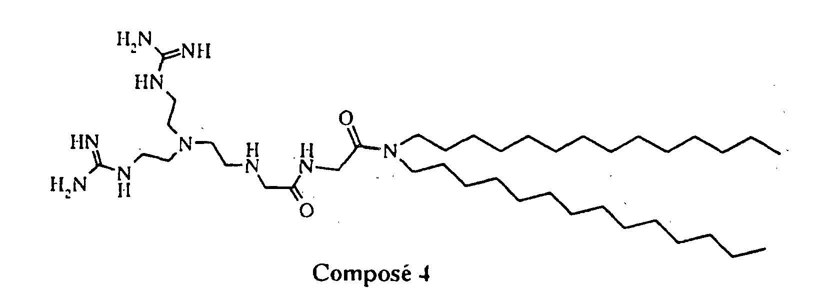

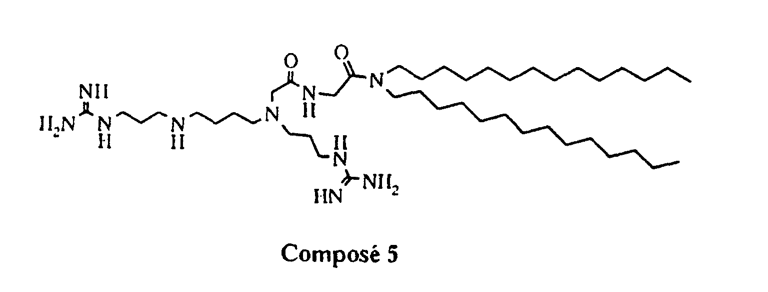

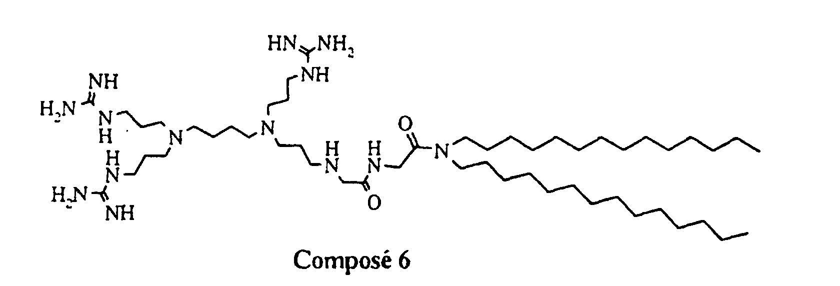

Les composés de l'invention peuvent être préparés de différentes façons, notamment par synthèse en solution et/ou par synthèse en phase solide sur un support polymérique.

Selon une première voie de synthèse, les composés de l'invention de formule générale (I) peuvent être obtenus par action d'un dérivé thio ou oxo de l'urée, dont les amines sont éventuellement protégées, sur une lipopolyamine de formule (VIII) : 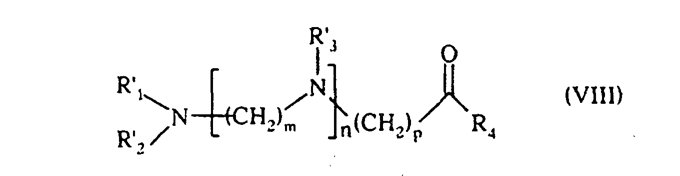 dans laquelle R'₁, R'₂, R'₃ représentent indépendamment les uns des autres un atome d'hydrogène ou un groupement (CH₂)_{q}-NR₉R₁₀ avec q pouvant varier entre 1 et 6 inclus, les différents q étant indépendants les uns des autres,
R₉ et R₁₀ représentent indépendamment l'un de l'autre un atome d'hydrogène ou un groupement de formule (CH₂)ᵣ-NH₂, r pouvant varier indépendamment les uns des autres entre 1 et 6 inclus,
et m, n, p et R₄ sont définis comme précédemment.

L'action du dérivé de l'urée s'effectue en général en présence d'une base, dans un solvant protique ou aprotique convenable, et à température comprise entre 0°C et 100°C.

La base utilisée est en général une base non-nucléophile comme par exemple les amines tertiaires, le carbonate de sodium ou le bicarbonate de sodium. Avantageusement, on utilise des amines tertiaires, par exemple la triéthylamine (TEA) ou la N-éthyldiisopropylamine (DIEA).

La réaction s'effectue avantageusement dans des solvants tels que l'eau, les alcools (méthanol, éthanol, isopropanol...), le diméthylformamide, le dichlorométhane, le chloroforme, le toluène, le tétrachlorure de carbone, le benzène, l'acétonitrile, le N-méthylpyrrolidone etc... Préférentiellement, la température de réaction est comprise entre 20°C et 60°C, et encore plus préférentiellement entre 30°C et 50 °C.

Des dérivés de l'urée plus particulièrement intéressants sont par exemple l'O-méthylisourée (J. Med. Chem., 38(1995) 16, 3053-3061), le semisulfate de S-méthylisothiourée (Int. J. Pept. Prot. Res., 40(1992), 119-126), la bis-Boc-thiourée (Tet. Lett. 48(1993), 7677-7680), la N,N'-bis(benzyloxycarbonyl)-S-méthylisothiourée ou la N,N'-bis(tert-butoxycarbonyl)-S- méthylisothiourée (Synth. Commun., 26(1996), 2, 407-413).

Les lipopolyamines de formule générale (VIII) sont obtenues selon les méthodes décrites dans la demande de brevet WO97/18185 incorporée à la présente par référence, ou par toute méthode analogue connue de l'homme du métier.

Selon l'invention, les agents de transfert de formule générale (I) peuvent aussi être obtenus par couplage peptidique entre l'acide de formule générale (IX) : 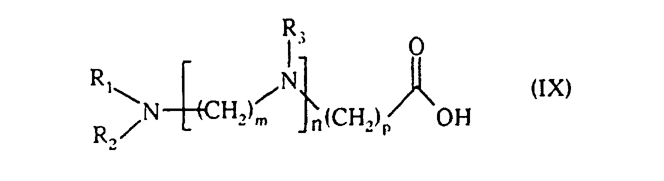 et la molécule lipidique R₄H pour lesquelles R₁, R₂, R₃, m, n, p et R₄ sont définis comme précédemment.

Le couplage peptidique s'effectue selon les méthodes classiques (Bodanski M., *Principles and Practices of Peptides Synthesis*, Ed. Springe-Verlag) ou par toute méthode analogue connue de l'homme du métier.

Notamment, la réaction s'effectue généralement en présence d'une base non-nucléophile dans des solvants aprotiques convenables, à température comprise entre 0 et 100°C, le pH étant ajusté entre 9 et 11.

A titre d'exemple, le chloroforme, le diméthylformamide, le N-méthylpyrrolidone, l'acétonitrile, le dichlorométhane, le toluène ou le benzène peuvent être utilisés comme solvant.

Les bases non-nucléophiles utilisées sont préférentiellement des amines tertiaires, du carbonate de calcium ou encore du dicarbonate de sodium. Encore plus préférentiellement, les bases utilisées sont des amines tertiaires, par exemple la triéthylamine (TEA) ou la N-éthyldiisopropylamine (DIEA).

Avantageusement, la réaction est effectuée à température comprise entre 0°C et 50°C, et plus préférentiellement entre 10°C et 30°C.

La molécule lipidique de formule R₄H, pour laquelle R₄ est défini comme précédemment, peut être obtenue par couplage peptidique entre le composé commercial de formule générale (X) : 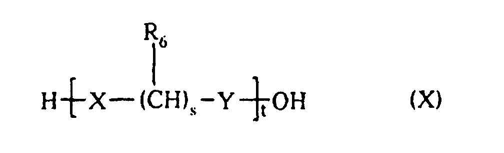 et l'amine de formule NHR₇R₈,
pour lesquelles X, Y, s, t, R₆, R₇ et R₈ sont définis comme précédemment.

Le couplage peptidique s'effectue selon les méthodes classiques (Bodanski M., *Principles and Practices of Peptides Synthesis*, Ed. Springe-Verlag) ou par toute méthode analogue connue de l'homme du métier.

Notamment, la réaction s'effectue généralement en présence d'une base non-nucléophile dans des solvants aprotiques convenables, à température comprise entre 0 et 100°C, le pH étant ajusté entre 9 et 11, comme cela a été décrit précédemment.
l'acide de formule générale (IX) peut être obtenu selon une voie de synthèse en phase solide en 3 étapes comme suit :
- Une polyamine de de formule générale (XI) : 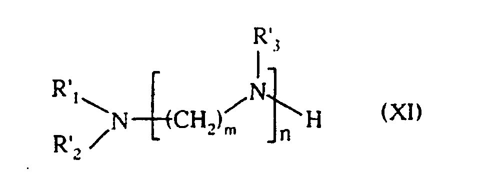 pour laquelle R'₁, R'₂, R'₃, m et n sont définis comme précédemment, est geffée sur un support polymérique 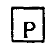 de façon à obtenir le composé gréffé de formule générale (XII) : 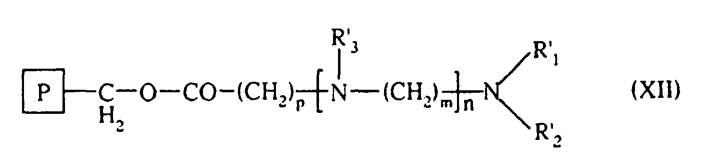
   La réaction s'effectue de préférence à température comprise entre 0°C et 100°C en présence d'un solvant aprotique convenable, par exemple le chloroforme, le dichlorométhane, le diméthylformamide, le N-méthylpyrrolidone, l'acétonitrile, le toluène, le benzène, etc... Avantageusement, la température de réaction est la température ambiante.
   Différents supports polymériques conviennent. On choisit avantageusement les résines disponibles dans le commerce pour la synthèse peptidique en phase solide (synthèse de Merrifield). Notamment, on peut choisir la résine de chlorure d'O-Chlorotrityle ou la résine HMP qui fournissent des produits portant des fonctions acides libres, ou une résine de type Rink. Les acides polyaminés peuvent être synthétisés directement sur un peptide présynthétisé sur la phase solide et portant une fonction bromoalkyle ou un acide w-aldéhyde.
   Les agents alkylants utilisés pour l'obtention de la résine appropriée sont choisis en fonction de la méthode d'alkylation. Pour une alkylation classique, on choisit par exemple l'acide bromoacétique ou les acides w-halogénocarboxyliques. Pour une alkylation réductive, on choisit par exemple un acide w-aldéhyde-carboxylique tel que l'acide glyoxalique, le semi-aldéhyde succinique etc..., ou un céto-acide tel que l'acide acéto-acétique ou l'acide pyruvique, etc...
   Les polyamines de départ de formule générale (XI) sont soit disponibles commercialement, par exemple les spermidine, spermine, tris-(2-aminoéthyle)amine, phénylène-diamine, diaminoéthane (propane, butane, pentane, hexane, etc...), soit peuvent être synthétisées par des méthodes classiques, par exemple par cyanoéthylation d'amines disponibles dans le commerce telles que les diaminoéthane (propane, butane, pentane, hexane, etc...) amine, spermidine, spermine, pour obtenir des polyamines branchées.
- Dans une seconde étape, on fait réagir le composé greffé de formule générale (XII) avec un dérivé thio ou oxo de l'urée, dont les amines sont éventuellement protégées, pour obtenir un composé polyaminoamidine greffé de formule générale (XIII) : 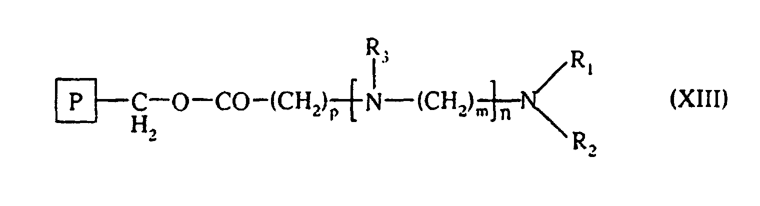
dans laquelle R₁, R₂, R₃, m, n et p sont définis comme précédemment.

La réaction s'effectue selon les méthodes connues de l'homme du métier (Bergeron, R.J. and McManis, *Total synthesis of 15-Deoxyspergualin*, J. Org. Chem., 1987, 52, 1700-1703) ou selon des méthodes analogues.

La réaction s'effectue notamment à température comprise entre -20°C et 100°C en présence d'un solvant protique ou aprotique.

A titre d'exemple, on utilise en tant que solvant l'eau, les alcools (méthanol, éthanol, isopropanol etc...), le diméthylformamide, le dichlorométhane, le chloroforme, les solvants aromatiques (toluène, benzène, etc...), le tétrachlorure de carbone, l'acétonitrile, le N-méthylpyrrolidone, etc...

Des dérivés de l'urée plus particulièrement intéressants sont par exemple l'O-méthylisourée (J. Med. Chem., 38(1995) 16, 3053-3061), le semisulfate de S-méthylisothiourée (Int. J. Pept. Prot. Res., 40(1992), 119-126), la bis-Boc-thiourée (Tet. Lett. 48(1993), 7677-7680), la N,N'-bis(benzyloxycarbonyl)-S-méthylisothiourée ou la N,N'-bis(tert-butoxycarbonyl)-S- méthylisothiourée (Synth. Commun., 26(1996), 2, 407-413).
- Enfin, dans une dernière étape, les polyaminoamidines greffées de formule générale (XIII) sont clivées du support polymérique pour donner l'acide de formule générale (IX) tel que défini précédemment. Le clivage est effectué selon les méthodes classiques connues de l'homme du métier. Notamment on opère par action d'un acide faible qui ne dégrade pas le reste de la molécule. Des acides faibles préférés sont par exemple les alcools fluorés, et plus particulièrement le 1,1,1-trifluoroéthan-2-ol.
   La polyaminoamidine de formule générale (XIII) contenant des fonctions acides, amino, alkylamino et/ou amidine, il est préférable le cas échéant de les protéger préalablement au clivage du support polymérique. La protection peut être réalisée par tout groupement compatible dont la mise en oeuvre et l'élimination n'altère pas le reste de la molécule. Notamment, on opère selon les méthodes décrites dans T.W. GREENE, *Protective Groups in Organic Synthesis*, A. Wiley-Interscience Publication (1981), ou dans McOmie, *Protective Groups in Organic Chemistry*, Plenum Press (1973).

L'élimination, le cas échéant, des radicaux protecteurs est effectuée préalablement au couplage peptidique entre l'acide de formule générale (IX) obtenu après clivage du support polymérique, et le composé de formule R₄H selon les méthodes habituelles connues de l'homme du métier.

A titre d'exemple, les groupements protecteurs peuvent être choisis parmi les radicaux triméthysilyte, benzhydryle, tétrahydropyrannyle, formyle, acétyle, chloracétyle, trichloracétyle, trifluoroacétyle, éthoxycarbonyle, tert-butoxycarbonyle, trichloroéthoxycarbonyle, benzyloxycarbonyl, fluorényloxycarbonyle, etc...

Toute autre méthode connue de l'homme du métier, et notamment celles décrites dans Bodanski M., *Principles and Practices of Peptides Synthesis*, Ed. Springe-Verlag, conduisant aux agents de transfert d'acides nucléiques selon la présente invention entre également dans le cadre de l'invention.

Un autre objet de l'invention concerne une composition comprenant un agent tel que défini ci-avant, et un acide nucléique. Préférentiellement, le composé et l'acide nucléique sont présents en quantités telles que le rapport R des charges positives du composé sur les charges négatives de l'acide nucléique soit compris entre 0,1 et 50, et plus préfrentiellement entre 0,1 et 20. Ce rapport peut être ajusté aisément par l'homme du métier en fonction du composé utilisé, de l'acide nucléique, et des applications recherchées (notamment du type de cellules à transfecter).

On entend au sens de l'invention par "acide nucléique" aussi bien un acide désoxyribonucléique qu'un acide ribonucléique. Il peut s'agir de séquences naturelles ou artificielles, et notamment d'ADN génomique (ADNg), d'ADN complémentaire (ADNc), d'ARN messager (ARNm), d'ARN de transfert (ARNt), d'ARN ribosomique (ARNr), de séquences hybrides ou de séquences synthétiques ou semi-synthétiques, d'oligonucléotides modifiés ou non. Ces acides nucléiques peuvent être d'origine humaine, animale, végétale, bactérienne, virale, etc... Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement.

Concernant plus particulièrement les acides désoxyribonucléiques, ils peuvent être simple ou double brin de même que des oligonuléotides courts ou des séquences plus longues. En particulier, les acides nucléiques sont avantageusement constitués par des plasmides, vecteurs, épisomes, cassettes d'expression, etc... Ces acides désoxyribonucléiques peuvent porter une origine de réplication fonctionnelle ou non dans la cellule cible, un ou plusieurs gènes marqueurs, des séquences régulatrices de la transcription ou de la réplication, des gènes d'intérêt thérapeutique, des séquences antisens modifiées ou non, des régions de liaison à d'autres composants cellulaires, etc...

De préférence, l'acide nucléique comprend une cassette d'expression constituée d'un ou plusieurs gènes d'intérêt thérapeutique sous contrôle d'un ou plusieurs promoteurs et d'un terminateur transcriptionnel actif dans les cellules cibles.

Au sens de l'invention, on entend par gène d'intérêt thérapeutique notamment tout gène codant pour un produit protéique ayant un effet thérapeutique. Le produit protéique ainsi codé peut être notamment une protéine ou un peptide. Ce produit protéique peut être exogène homologue ou endogène vis-à-vis de la cellule cible, c'est-à-dire un produit qui est normalement exprimé dans la cellule cible lorsque celle-ci ne présente aucune pathologie. Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène d'intérêt thérapeutique peut aussi coder pour un mutant d'une protéine cellulaire, ayant une stabilité accrue, une activité modifiée, etc... Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc... (FR 92/03120), les facteurs de croissance, les neurotransmetteurs ou leurs précurseurs ou enzymes de synthèse, les facteurs trophiques (BDNF, CNTF, NGF, IGF, GMF, aFGF, bFGF, NT3, NT5, HARP/pléiotrophine, etc...) les apolipoprotéines (ApoAI, ApoAIV, ApoE, etc..., FR 93/05125), la dystrophine ou une minidystrophine (FR 91/11947), la protéine CFTR associée à la mucoviscidose, les gènes suppresseurs de tumeurs (p53, Rb, RaplA, DCC, k-rev, etc..., FR 93/04745), les gènes codant pour des facteurs impliqués dans la coagulation (Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les enzymes du métabolisme, catabolisme etc...

L'acide nucléique d'intérêt thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

L'acide nucléique peut aussi comporter un ou plusieurs gènes codant pour un peptide antigénique, capable de générer chez l'homme ou l'animal une réponse immunitaire. Dans ce mode particulier de mise en oeuvre, l'invention permet la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore de peptides antigéniques spécifiques de tumeurs (EP 259 212).

Préférentiellement, l'acide nucléique comprend également des séquences permettant l'expression du gène d'intérêt thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exempte, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc... En outre, ces séquences d'expression peuvent être modifiées par addition de séquences d'activation, de régulation, etc... Il peut aussi s'agir de promoteur, inductible ou répressible.

Par ailleurs, l'acide nucléique peut également comporter, en particulier en amont du gène d'intérêt thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment cellulaire particulier.

Dans un autre mode de mise en oeuvre, la présente invention concerne également des compositions comprenant un acide nucléique, un agent de transfection (I) tel que défini ci-avant et un ou plusieurs adjuvants capables de s'associer au complexe agent de transfection/acide nucléique et d'en améliorer le pouvoir transfectant. La présence de ce type d'adjuvants (lipides, peptides ou protéines par exemple) peut permettre avantageusement d'augmenter le pouvoir transfectant des composés.

Dans cette optique, les compositions de l'invention peuvent comprendre comme adjuvant, un ou plusieurs lipides neutres. De telles compositions sont particulièrement avantageuses, notamment lorsque le rapport de charge R des complexes nucléolipidiques est faible. La demanderesse a en effet montré que l'addition d'un lipide neutre permet d'améliorer la formation des particules nucléolipidiques et de favoriser la pénétration de la particule dans la cellule en déstabilisant sa membrane.

Plus préférentiellement, les lipides neutres utilisés dans le cadre de la présente invention sont des lipides à 2 chaînes grasses. De manière particulièrement avantageuse, on utilise des lipides naturels ou synthétiques, zwitterioniques ou dépourvus de charge ionique dans les conditions physiologiques. Il peuvent être choisis plus particulièrement parmi la dioléoylphosphatidyléthanolamine (DOPE), l'oléoylpatmitoylphosphatidyléthanolamine (POPE), les di-stéaroyl, -palmitoyl,-mirystoylphosphatidyléthanolamines ainsi que leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels que par exemple les sphingomyélines), ou encore les asialogangliosides (tels que notamment les asialoGM1 et GM2).

Ces différents lipides peuvent être obtenus soit par synthèse, soit par extraction à partir d'organes (par exemple le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Plus récemment, la demanderesse a démontré qu'il était également particulièrement avantageux d'employer à titre d'adjuvant, un composé intervenant ou non directement au niveau de la condensation dudit acide nucléique (WO 96/25508). La présence d'un tel composé, au sein d'une composition selon l'invention permet de diminuer la quantité de composé transfectant, avec les conséquences bénéfiques qui en découlent sur le plan toxicologique, sans porter un préjudice quelconque à l'activité transfectante. Par composé intervenant au niveau de la condensation de l'acide nucléique, on entend définir un composé compactant, directement ou non, l'acide nucléique. Plus précisément, ce composé peut soit agir directement au niveau de l'acide nucléique à transfecter soit intervenir au niveau d'un composé annexe qui lui est directement impliqué dans la condensation de cet acide nucléique. De préférence, il agit directement au niveau de l'acide nucléique. Par exemple, l'agent précompactant peut être tout polycation, et par exemple la polylysine. Selon un mode de réalisation préféré, cet agent intervenant au niveau de la condensation de l'acides nucléique dériv en tout ou en partie d'une protamine, d'une histone, ou d'une nucléoline et/ou de l'un de leurs dérivés. Un tel agent peut également être constitué, en tout ou partie, de motifs peptidiques (KTPKKAKKP) et/ou (ATPAKKAA), le nombre de motifs pouvant varier entre 2 et 10. Dans la structure du composé selon l'invention, ces motifs peuvent être répétés de manière continue ou non. C'est ainsi qu'ils peuvent être séparés par des liens de nature biochimique, par exemple par un ou plusieurs acides aminés, ou de nature chimique.
Préférentiellement, les compositions de l'invention comprennent de 0,01 à 20 équivalents d'adjuvant pour un équivalent d'acides nucléiques en mol/mol et, plus préférentiellement, de 0,5 à 5.

Dans un mode de réalisation particulièrement avantageux, les compositions de la présente invention comprennent en outre un élément de ciblage permettant d'orienter le transfert de l'acide nucléique. Cet élément de ciblage peut être un élément de ciblage extracellulaire permettant d'orienter le transfert de l'ADN vers certains, types cellulaires ou certains tissus souhaités (cellules tumorales, cellules hépatiques, cellules hématopoiétiques...). Il peut également s'agir d'un élément de ciblage intracellulaire permettant d'orienter le transfert de l'acide nucléique vers certains compartiments cellulaires privilégiés (mitochondries, noyau etc...). L'élément de ciblage peut être lié à l'agent de transfert d'acides nucléiques selon l'invention ou également à l'acide nucléique comme cela a été précisé précédemment.

Parmi les éléments de ciblage utilisables dans le cadre de l'invention, on peut citer les sucres, les peptides, les protéines, les oligonucléotides, les lipides, les neuromédiateurs, les hormones, les vitamines ou leurs dérivés. Préférentiellement, il s'agit de sucres de peptides ou de protéines tels que des anticorps ou des fragments d'anticorps, des ligands de récepteurs cellulaires ou des fragments de ceux-ci, des récepteurs ou des fragments de récepteurs, etc... En particulier, il peut s'agir de ligands de récepteurs de facteurs de croissance, de récepteurs de cytokines, de récepteurs de type lectines cellulaires, ou de ligands à séquence RGD avec une affinité pour les récepteurs de protéines d'adhésion comme les intégrines. On peut également citer les récepteurs de la transferrine, des HDL et des LDL, ou le transporteur du folate. L'élément de ciblage peut également être un sucre permettant de cibler des lectines tels que les récepteurs aux asialoglycoprotéines ou aux syalydés tel que le sialyde Lewis X, ou encore un fragment Fab d'anticorps, ou un anticorps simple chaîne (ScFv).

L'association des éléments de ciblage aux complexes nucléolipidiques peut être effectuée par toute technique connue de l'homme du métier, par exemple par couplage à une partie hydrophobe ou à une partie qui interagit avec l'acide nucléique de l'agent de transfert selon l'invention, ou encore à un groupement qui interagit avec l'agent de transfert selon l'invention ou avec l'acide nucléique. Les interactions en question peuvent être, selon un mode préféré, de nature ionique ou covalente.

L'invention a également pour objet l'utilisation des composés tels que définis ci-avant pour le transfert d'acides nucléiques (et plus généralement de polyanions) dans les cellules.

Pour des utilisations *in vivo*, par exemple pour l'étude de la régulation de gènes, la création de modèles animaux de pathologies, ou en thérapie, les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, intratrachéale, intrapéritonéale, etc... De préférence, les compositions de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus, par exemple au niveau des tumeurs, ou les voies circulatoires, soit d'un traitement de cellules en culture suivi de leur réimplantation *in vivo*, par injection ou greffe. Les tissus ou voies circulatoires concernés dans le cadre de la présente invention sont par exemple les muscles, la peau, le cerveau, les poumons, le foie, la rate, la moelle osseuse, le thymus, le coeur, la lymphe, le sang, les os, les cartilages, le pancréas, les reins, la vessie, l'estomac, les intestins, les testicules, les ovaires, le rectum, le système nerveux, les yeux, les glandes, les tissus conjonctifs, etc...

L'invention concerne en outre une méthode de transfert d'acides nucléiques dans les cellules comprenant les étapes suivantes :
(1) la mise en contact de l'acide nucléique avec un agent de transfert tel que défini ci-avant, pour former un complexe et,
(2) la mise en contact des cellules avec le complexe formé en (1).

La mise en contact des cellules avec le complexe peut être réalisée par incubation des cellules avec ledit complexe (pour des utilisations *in vitro* ou *ex vivo),* ou par injection du complexe dans un organisme (pour des utilisations *in vivo*). L'incubation est réalisée de préférence en présence de par exemple de 0,01 à 1000 µg d'acide nucléique pour 10⁶ cellules. Pour une administration *in vivo*, des doses d'acide nucléique comprises entre 0,01 et 10 mg peuvent par exemple être utilisées.

Dans le cas où les compositions selon la présente invention contiennent en outre un ou plusieurs adjuvants et/ou un élément de ciblage tels que définis précédemment, le ou les adjuvants et/ou l'élément de ciblage sont préalablement mélangés à l'agent de transfert selon l'invention ou à l'acide nucléique.

La présente invention fournit ainsi une méthode particulièrement avantageuse pour le transfert d'acides nucléiques, notamment pour le traitement de maladies, comprenant l'administration *in vitro*, *in vivo* ou *ex vivo* d'un acide nucléique apte à corriger ladite maladie associé à un agent de transfert de formule générale (I) dans les conditions définies ci-avant. Plus particulièrement; cette méthode est applicable aux maladies résultant d'une déficience en un produit protéique ou nucléique, l'acide nucléique administré codant pour le dit produit protéique ou étant transcrit en un produit nucléique, ou encore constituant le dit produit nucléique.

La présente invention s'étend également à toute utilisation d'un agent de transfert de formule générale (I) selon l'invention pour la transfection *in vivo*, *ex vivo* ou *in vitro* de cellules.

Les composés de l'invention sont en particulier utilisables pour le transfert d'acides nucléiques dans des cellules primaires ou dans des lignées établies. Il peut s'agir de cellules fibroblastiques, musculaires, nerveuses (neurones, astrocytes, cellules gliales), hépatiques, de la lignée hématopoiétique (lymphocytes, CD34, dendritiques, etc...), épithéliales, etc..., sous forme différenciées ou pluripotentes (précurseurs).

Outre les dispositions qui précèdent, la présente invention comprend également d'autres caractéristiques et avantages qui ressortiront des exemples et figures qui suivent, et qui doivent être considérés comme illustrant l'invention sans en limiter la portée.

### FIGURES

Figure 1/15 : schéma de synthèse du composé 1 selon l'invention.
Figure 2/15 : mesure de l'efficacité de transfection *in vitro* du composé 1 selon l'invention dans les cellules NIH3T3, HepG2 et HeLa, en l'absence de protéines sériques et comparativement à son homologue aminé (RPR120535)
   On entend par "homologue aminé" le lipide cationique identique à l'exception des fonctions amidine et/ou guanidine qui sont remplacées par des fonctions amino.
   Les mesures ont été faites à différents rapports de charge qui sont portés en absisse. L'expression de la luciférase est portée sur l'axe des ordonnées et est exprimée en RLU (Relative Light Unit) par puit.

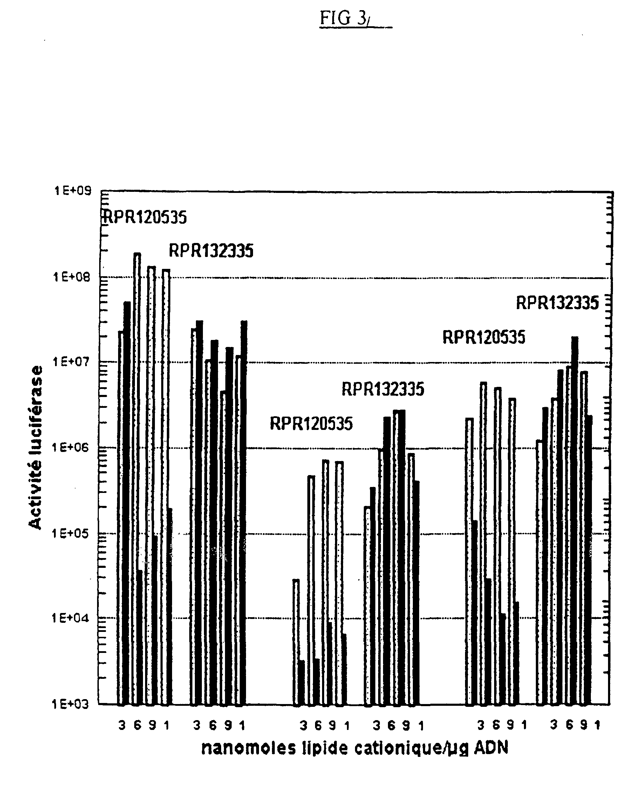

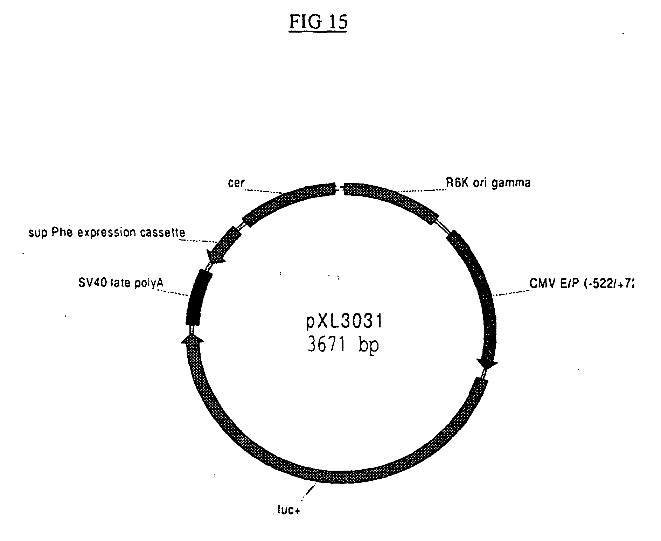

Figure 3/15 : mesure de l'efficacité de transfection *in vitro* du composé 1 selon l'invention dans les cellules NIH3T3, HepG2 et HeLa, en l'absence (barres blanches) et en présence (barres noires) de protéines sériques et comparativement à son homologue aminé (RPR120535).
   Les mesures ont été faites à différents rapports de charge qui sont portés en absisse. L'expression de la luciférase est portée sur l'axe des ordonnées et est exprimée en RLU (Relative Light Unit) par puit.

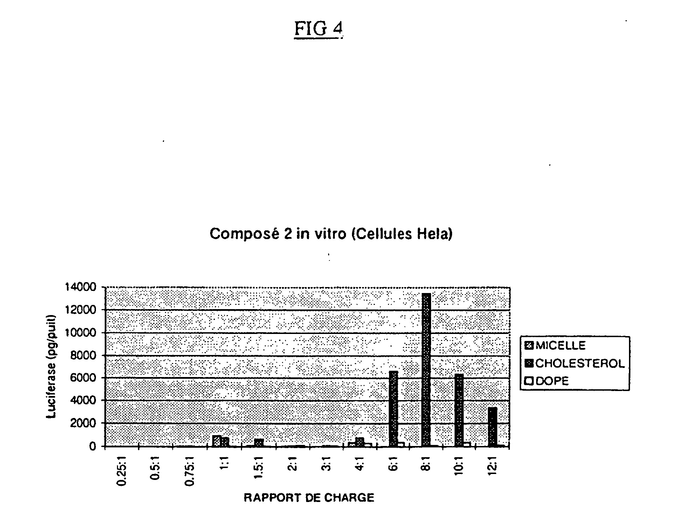

Figure 4/15 : mesure de l'efficacité de transfection *in vitro* du composé 2 selon l'invention dans les cellules HeLa, en l'absence de protéines sériques. Les mesures ont été faites pour le composé 2 sous forme micellaire et en association avec un co-lipide neutre (DOPE et cholestérol).
   L'axe des absisses représente le rapport de charge du complexe formé entre le lipide cationique et l'ADN. L'axe des ordonnées représente l'expression de la luciférase exprimée en pg/puit, chaque puit contenant 100 000 cellules.

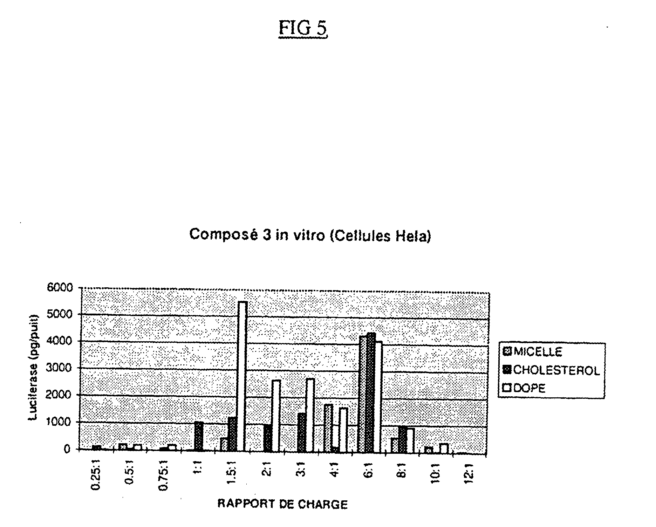

Figure 5/15 : mesure de l'efficacité de transfection *in vitro* du composé 3 selon l'invention dans les cellules HeLa, en l'absence de protéines sériques. Les mesures ont été faites pour le composé 3 sous forme micellaire et en association avec un co-lipide neutre (DOPE et cholestérol).
   L'axe des absisses représente le rapport de charge du complexe formé entre le lipide cationique et l'ADN. L'axe des ordonnées représente l'expression de la luciférase exprimée en pg/puit, chaque puit contenant 100 000 cellules.

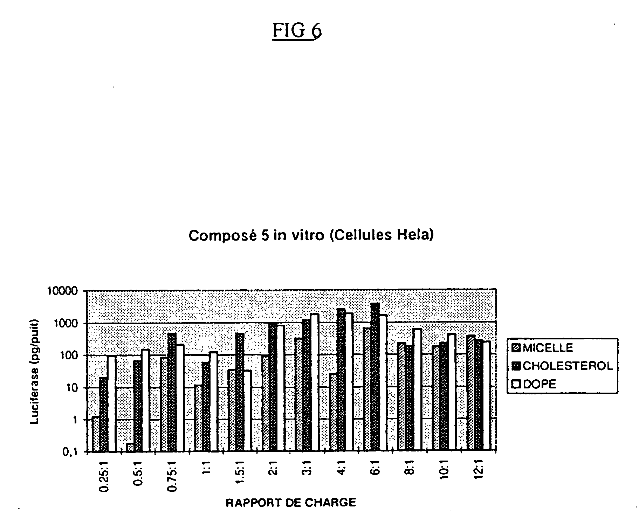

Figure 6/15 : mesure de l'efficacité de transfection *in vitro* du composé 5 selon l'invention dans les cellules HeLa, en l'absence de protéines sériques. Les mesures ont été faites pour le composé 5 sous forme micellaire et en association avec un co-lipide neutre (DOPE et cholestérol).
   L'axe des absisses représente le rapport de charge du complexe formé entre le lipide cationique et l'ADN. L'axe des ordonnées représente l'expression de la luciférase exprimée en pg/puit, chaque puit contenant 100 000 cellules.

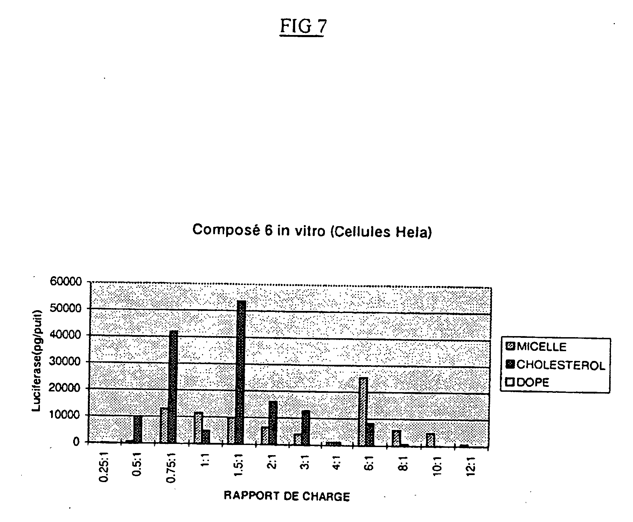

Figure 7/15 : mesure de l'efficacité de transfection *in vitro* du composé 6 selon l'invention dans les cellules HeLa, en l'absence de protéines sériques. Les mesures ont été faites pour le composé 6 sous forme micellaire et en association avec un co-lipide neutre (DOPE et cholestérol).
   L'axe des absisses représente le rapport de charge du complexe formé entre le lipide cationique et l'ADN. L'axe des ordonnées représente l'expression de la luciférase exprimée en pg/puit, chaque puit contenant 100 000 cellules.

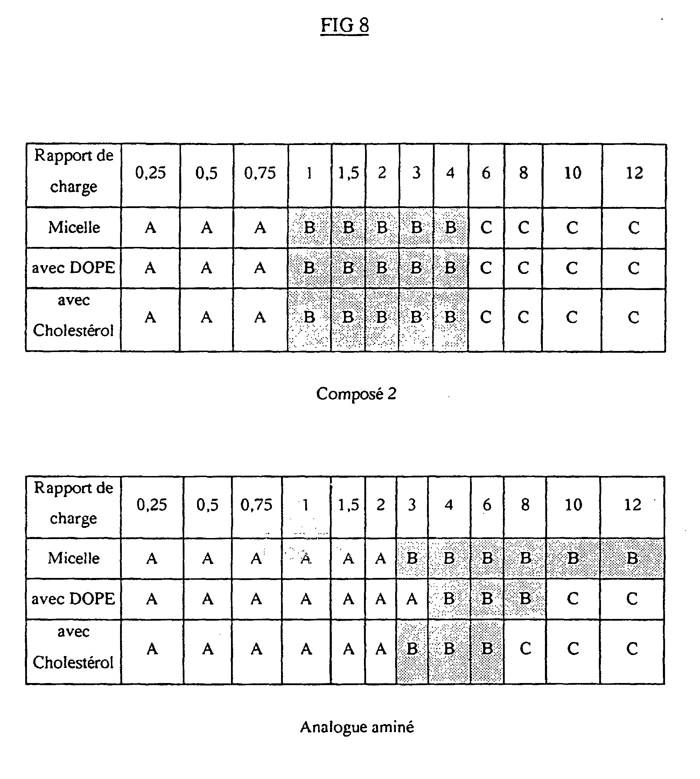

Figure 8/15 : tableaux indiquant la phase physico-chimique (phase A, B ou C) dans laquelle se trouvent les complexes nucléolipidiques formés avec le composé 2 comparativement à son analogue aminé, en fonction du rapport de charge. La détermination des phases a été faite pour le composé 2 et pour son homologue aminé sous forme micellaire et en association avec un co-lipide neutre (DOPE et cholestérol).
   En comparant les 2 tableaux, on observe un décalage de la phase B instable vers des rapports de charge plus bas dans le cas du composé 2.

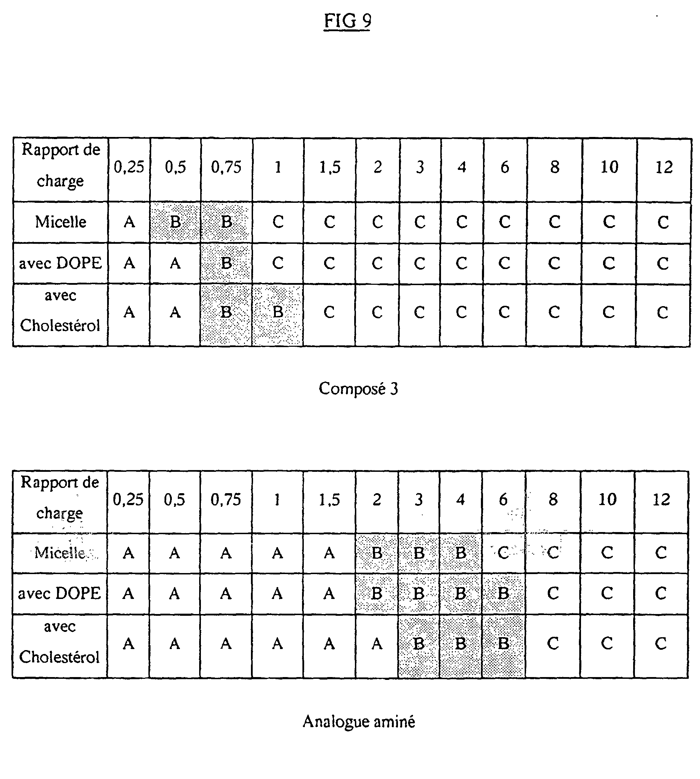

Figure 9/15 : tableaux indiquant la phase physico-chimique (phase A, B ou C) dans laquelle se trouvent les complexes nucléolipidiques formés avec le composé 3 comparativement à son analogue aminé, en fonction du rapport de charge.
   La détermination des phases a été faite pour le composé 3 et pour son homologue aminé sous forme micellaire et en association avec un co-lipide neutre (DOPE et cholestérol).
   En comparant les 2 tableaux, on observe un décalage de la phase B instable vers des rapports de charge plus bas dans le cas du composé 3.

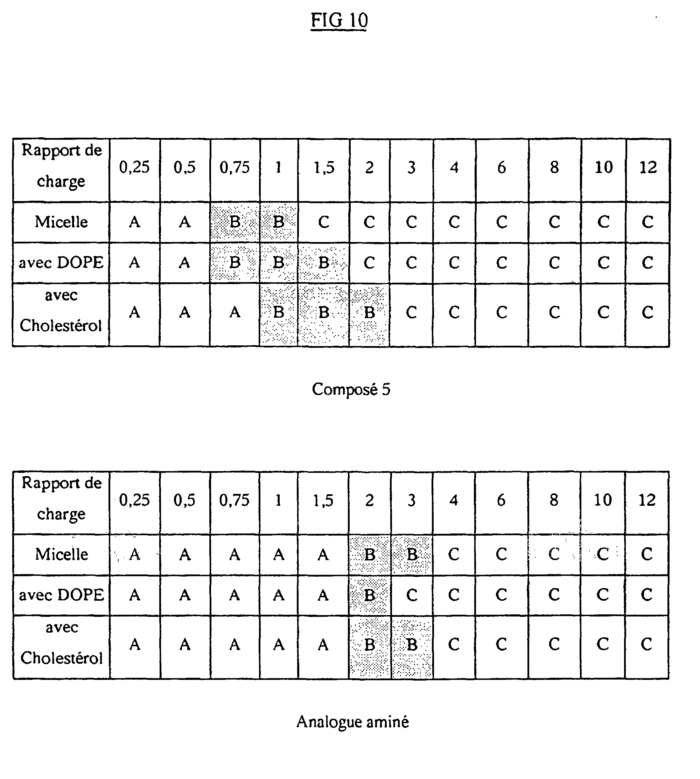

Figure 10/15 : tableaux indiquant la phase physico-chimique (phase A, B ou C) dans laquelle se trouvent les complexes nucléolipidiques formés avec le composé 5 comparativement à son analogue aminé, en fonction du rapport de charge.
   La détermination des phases a été faite pour le composé 5 et pour son homologue aminé sous forme micellaire et en association avec un co-lipide neutre (DOPE et cholestérol).
   En comparant les 2 tableaux, on observe un décalage de la phase B instable vers des rapports de charge plus bas dans le cas du composé 5.

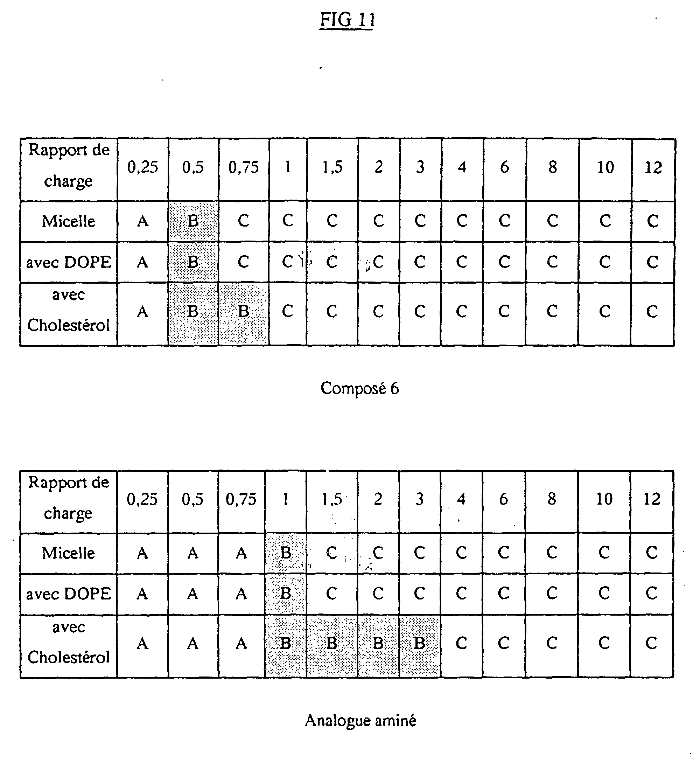

Figure 11/15 : tableaux indiquant la phase physico-chimique (phase A, B ou C) dans laquelle se trouvent les complexes nucléolipidiques formés avec le composé 6 comparativement à son analogue aminé, en fonction du rapport de charge.
   La détermination des phases a été faite pour le composé 6 et pour son homologue aminé sous forme micellaire et en association avec un co-lipide neutre (DOPE et cholestérol).
   En comparant les 2 tableaux, on observe un décalage de la phase B instable vers des rapports de charge plus bas dans le cas du composé 6.

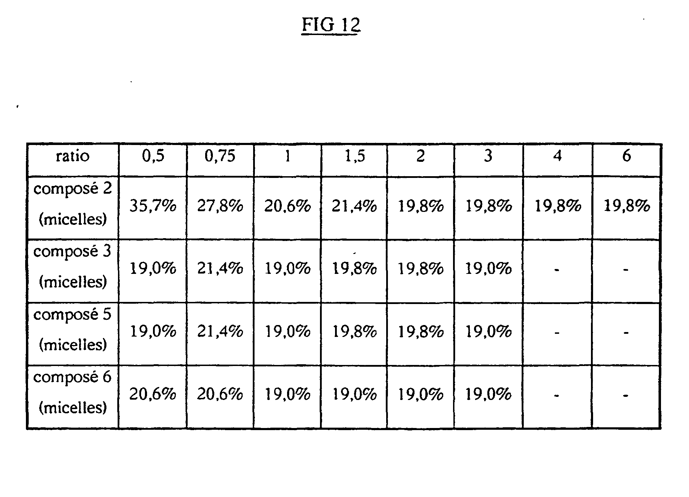

Figure 12/15 : tableau représentant la réduction de fluorescence après ajout de bromure d'éthidium pour les composés 2, 3, 5 et 6 sous forme de micelles. La substitution du bromure d'éthidium de l'ADN par le lipide est une indication de liaison à l'ADN. La fluorescence obtenue pour l'ADN seul est défini comme étant de 100%.

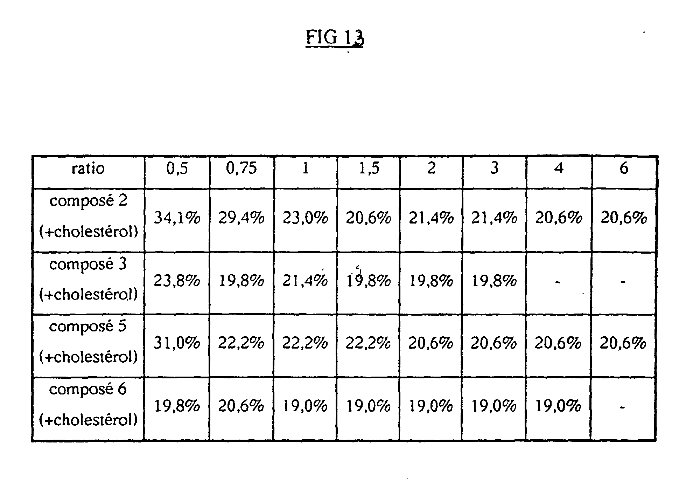

Figure 13/15 : tableau représentant la réduction de fluorescence après ajout de bromure d'éthidium pour les composés 2, 3, 5 et 6 en présence de cholestérol. La substitution du bromure d'éthidium de l'ADN par le lipide est une indication de liaison à l'ADN. La fluorescence obtenue pour l'ADN seul est défini comme étant de 100%.

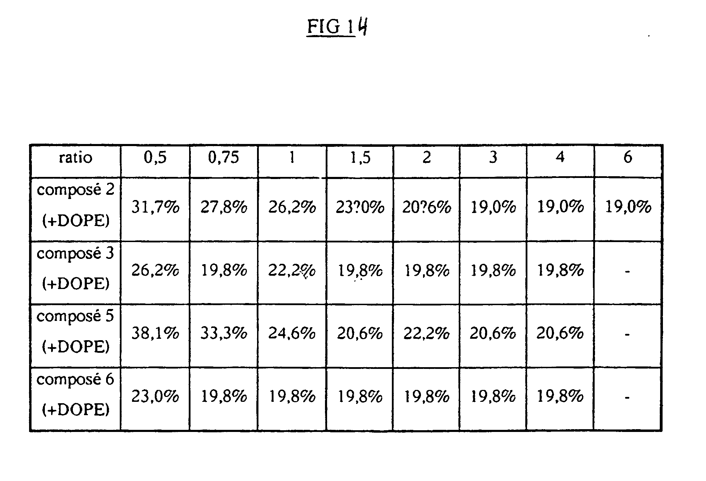

Figure 14/15 : tableau représentant la réduction de fluorescence après ajout de bromure d'éthidium pour les composés 2, 3, 5 et 6 en présence de DOPE. La substitution du bromure d'éthidium de l'ADN par le lipide est une indication de liaison à l'ADN. La fluorescence obtenue pour l'ADN seul est défini comme étant de 100%.
Figure 15/15 : représentation du plasmide pXL3031.

### ABREVIATIONS ET SYMBOLES

- AcOEt :: Acétate d'éthyle
- BOC :: Tert-butoxycarbonyle
- BOP :: Benzotriazol-1-yloxytris(diméthylamino)phosphonium hexafluorophosphate
- DCC :: Dicyclohexylcarbodiimide
- DCU :: Dicyclohexylurée
- DIEA :: N-Ethyldiisopropylamine
- DMAP :: 4-Diméthylaminopyridine
- DM F :: Diméthylformamide
- DMSO :: Diméthylsulfoxide
- DODA :: Dioctadécylamine
- EP :: Ether de pétrole
- EtOH :: Ethanol
- NEt3 :: Triéthylamine
- Rf :: Coefficient de rétention frontal
- TEA :: Triéthylamine
- TFA :: Acide trifluoroacétique
- THF :: Tétrahydrofurane
- TMS :: Tétraméthylsilane
- UV :: Ultra-Violets
- SPPS :: Synthèse Peptidique en Phase Solide(Solid Phase Peptide Synthesis)
- CLHP :: Chromatographie Liquide à Haute Pression
- Z :: Benzyloxycarbonyle
- CIZ:: p-chlorobenzyloxycarbonyle

### MATERIELS ET METHODES POUR LES SYNTHESES CHIMIOUES

### a) Composés

- Les polyamines de départ sont disponibles commercialement, par exemple les spermidine, spermine, tris-(2-aminoéthyle)amine, phénylène-diamine, diaminoéthane (propane, butane, pentane, hexane, etc...), ou peuvent être synthétisées par des méthodes classiques, par exemple par cyanoéthylation d'amines disponibles dans le commerce telles que les diaminoéthane (propane, butane, pentane, hexane, etc.) amine, spermidine, spermine, pour obtenir des polyamines branchées.
- Les polymères utilisés sont des résines disponibles dans le commerce pour la synthèse peptidique en phase solide (synthèse de Merrifield), notamment la résine d'O-Chlorotrityl chlorure, la résine HMP, qui fournissent des produits portant des fonctions acides libres, ou une résine de type Rink. Les acides polyaminés peuvent être synthétisés directement sur un peptide présynthétisé sur la phase solide et portant une fonction bromoalkyle ou un acide w-aldéhyde.
- Les dioctadécylamine, triéthylamine trifluoroacétique, BOP, DMAP, chloroformate de benzyle sont des produits commerciaux. Les solutions de NaCl et NaHCO₃ sont saturées; la solution de KHSO₄ est à 0,5 M.

### b) Mesures Physiques.

Les spectres de RMN du proton ont été enregistrés sur des spectrométres Bruker 400 et 600 MHZ.

Les spectres de masse (MS) ont été réalisés sur un API-MS/III.

### c) Techniques de purification et d'analyse

### a) Conditions de chromatographie en phase directe

- Les chromatographies sur couche mince (CCM) ont été effectuées sur des plaques de gel de silice Merck de 0,2 mm d' épaisseur.
   Elles sont révélées soit aux UV (254 nm), à la ninhydrine, en vaporisant (spray léger) une solution éthanolique de ninhydrine (40 mg/100 ml d'EtOH) pour révéler les amines ou les amides en chauffant à 150°C, à la fluorescamine en vaporisant une solution (40 mg/100 ml d'acétone) pour révéler les amines primaires, ou à l'iode en recouvrant la plaque de poudre d'iode.
- Les chromatographies sur colonne en phase directe ont été effectuées sur gel de silice 60 Merck de granulométrie 0,063-0,200 mm.

### b) Techniques de chromatographie analytique

Les analvses CLHP (Chromatographie Liquide Haute performance) ont été réalisées sur un appareil Merck-Hitachi équipé d'un intégrateur-calculateur D 2500 HITACHI, d'un autosampler AS-2000A, d'une pompe inteligent L-6200A, et d'un détecteur UV-vis L-4000 avec longueur d'onde réglable mise à 220 nm pour les séparations analytiques. Les colonnes pour les séparations analytiques sont des colonnes BU-300 aquapore Butyl 7m, 300 A 300x4,6 mm de chez Perkin-Elmer. Les phases mobiles sont de l'eau déminéralisée contenant 0,1 % de TFA et de l'acétonitrile contenant 0,1% de TFA. Les injection d'une solution d'environ 1 mg/ml dans une vanne à boucle de 100 µl sont de 20 µl. Le débit pour les analyses est réglé à 1 ml/min.

| Conditions de separation : | | | |
|---|---|---|---|
| Solvant A | | Solvant B | |
| Eau déminéralisée | 2500 ml | Acétonitrile pour HPLC | 2500 ml |
| Acide trifluoroacétique | 2 ml | Acide trifluoroacétique | 2.5 ml |

| Gradient : | | | |
|---|---|---|---|
| Temps en minutes | % de solvant A | % de solvant B | Débit en ml/minute |
| 0 | 60 | 40 | 1 |
| 3 | 60 | 40 | 1 |
| 20 | 0 | 100 | 1 |
| 35 | 0 | 100 | 1 |
| 35,1 | 60 | 40 | 4 |
| 36,1 | 60 | 40 | 4 |
| 36,2 | 60 | 40 | 2 |
| 44 | 60 | 40 | 2 |

### c) Techniques de chromatographie préparative

L'appareillage est un ensemble pour la chromatographie en phase liquide en mode gradient, permettant une détection UV. Cette chaîne préparative est composée des éléments suivants :
Pompe A : GILSON modèle 305 équipée d'une tête 50 SC.
Pompe B : GILSON modèle 303 équipée d'une tête 50 SC.
Boucle d'injection : GILSON modèle 303 équipée d'une tête 25 SC.
Module de pression : GILSON modèle 806.
Mélangeur : GILSON modèle 811 C équipé d'une tête de 23 ml.
Détecteur UV : GILSON modèle 119 équipé d'une cellule préparative.
Collecteur de fraction : GILSON modèle 202 équipé de portoirs n° 21.
Intégrateur : SHIMADZU modèle C-R6A.
Colonne : Colonne C4 (10 mm) en acier inoxydable de 25 cm de longueur et de 2,2 cm de diamètre commercialisée par VYDAC modèle 214 TP 1022.

La solution de produit à purifier est chargée sur la colonne par la pompe d'injection au débit de 15 ml/min, et l'éluat est receuilli par fraction d'un tube en 30 secondes. Le détecteur est réglé aux longueurs d'onde de 220 nm et 235 nm.

Les phases mobiles sont définies de la façon suivante :

| Solvant A | | Solvant B | |
|---|---|---|---|
| Eau déminéralisée | 2500 ml | Acétonitrile pour HPLC | 2500 ml |
| Acide trifluoroacétique | 2 ml | Acide trifluoroacétique | 2.5 ml |

| Gradient : | | | |
|---|---|---|---|
| Temps en minutes | % de solvant A | % de solvant B | Débit en ml/minute |
| 0 | 70 | 30 | 18 |
| 10 | 70 | 30 | 18 |
| 80 . | 0 | 100 | 18 |
| 120 | 0 | 100 | 18 |

### d) Technique de synthèse peptidique en phase solide (SPPS)

La synthèse en phase solide est réalisée dans un réacteur manuel de synthèse de peptides SPPS de fabrication artisanale, et l'agitateur est un Flask Shaker modèle A5-6021. L'évolution du couplage des polyamines à la phase solide ainsi que l'évolution de la protection des polyamines dans la SPPS est suivie par le test de Kaiser [Kaiser, E., Colescolt, D.L., Bossinger, C.D. and Cook, P.I. *Anal. Biochem*. 34(2), 595 (1970)]. La résine utilisée dans les exemples pour la SPPS est la "Chlorotrityl chloride Resin" de chez NOVABIOCHEM.

### EXEMPLES

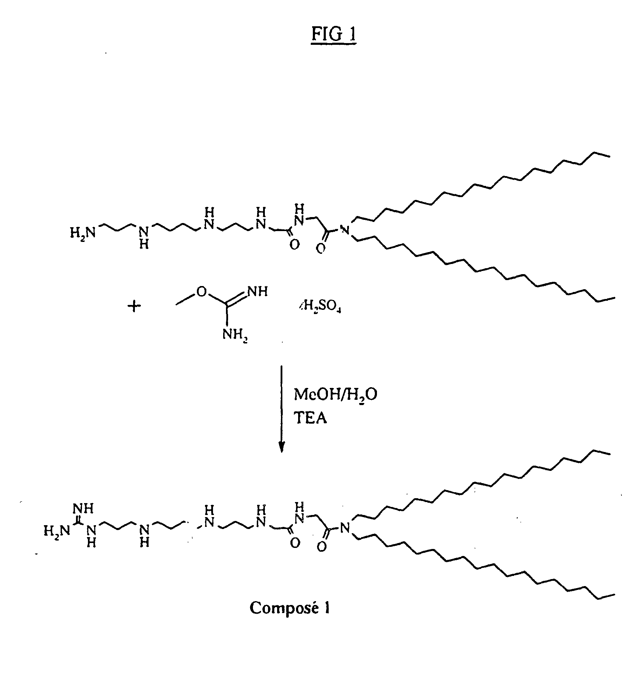

**Exemple 1 : Synthèse du composé 1** (N-dioctadécylcarbamoylméthyl-2-{3-[4-(3-guanidino-propylamino)-butylamino]-propylamino}-acétamide) à partir du composé RPR 120535 (dont la préparation a été décrite dans la demande de brevet WO 97/18185 incorporée à la présente par référence).
L'étape de synthèse est représentée à la figure 1/15 des dessins.
0,784 mmol de RPR 120535 sont dissoutes dans 25 ml de méthanol dans un ballon équipé d'un barreau magnétique. On ajoute à cette solution 10,21 mmol de triéthylamine TEA. Puis, on coule lentement (5 minutes) sur le mélange 1,173 mmol d'une solution de O-méthylisourée/acide sulfurique H₂SO₄ dans 9 ml d'eau. En fin de coulée, un trouble apparaît. Le mélange est maintenu à 40°C pendant 16 heures, puis la solution est évaporée à sec. Le produit obtenu est ensuite purifié par HPLC préparative. Les fractions intéressantes sont regroupées et lyophilisées.
On obtient 0,157 mmol de composé 1 salifié, soit un rendement de 20,1%.
HPLC_{analytique} : Rt = 14,94 min.
Spectre de RMN ¹H (400 MHz, (CD₃)₂SO d6, δ en ppm) : 0,86 (t, J= 7 Hz, 6H : CH₃ des chaînes grasses) ; 1,24 (mt, 60H : (CH₂)₁₅ centraux des chaînes grasses) ; 1,43 et 1,53 (2 mts, 2H chacun : 1 CH₂ de chaque chaîne grasse) ; 1,63 (mt, 4H : (CH₂)₂ centraux du butyle) ; 1,81 et 1,96 (2 mts, 2H chacun : CH₂ central des propyles) ; de 2,85 à 3,10 et 3,22 (2 mts, 16H en totalité : NCH₂ du butyle - NCH₂ des propyles et NCH₂ des chaînes grasses) ; 3,81 (s large, 2H : NCH₂CON) ; 4,03 (d, J = 4,5 Hz, 2H : CONCH₂CON du glycyle) ; 7,32 - 7,97 - 8,62 - 8,75 et 9,02 (respectivement mf - t - t - mf et mf : H correspondant aux protons échangeables).
MH⁺= 863

**Exemple 2 : Synthèse du composé 2** (2-{2-[bis-(2-guanidino-éthyl)-amino]-éthylamino}-N,N-dioctadécyl-acétamide) à partir du composé RPR 120527 (dont la préparation a été décrite dans la demande de brevet WO 97/18185 incorporée à la présente par référence).
48 µmol de RPR 120527 sont dissoutes dans 10 ml de méthanol, puis on ajoute 85 µl de DIEA (10 équivalents) et 32 mg de 1,3-bis-(tert-butoxycarbonyl)-2-méthyl-2-thiopseudourée (2,3 équivalents). La réaction est suivie par CLHP. Après 24 heures, le solvant est évaporé, et on ajoute au résidu obtenu 20 ml d'une solution TFA/dichlorométhane 1:1 en volume. Après évaporation sous vide, on purifie le résidu par CLHP préparative. Les fractions intéressantes sont mélangées, congelées dans l'azote liquide, puis lyophilisées.
On obtient ainsi 0,035 mmol de composé 2, soit un rendement de 73%.
HPLC analytique : Rt = 16,20 min.
Spectre de RMN ¹H (400 MHz, (CD₃)₂SO d6, δ en ppm): 0,9 (m : 6H : CH₃) 1,2 (m : 60H : CH₂) 1,6 (m : 4H : CH₂CH₂N) 2,7-2,9 (m : 6H : CH₂N(CH₂)₂) 3,0 (m : 2H : CH₂CH₂N(CH₂)₂) 3,2 (m : 8H : CH₂N) 3,8 (m : 4H : CH₂NCOCH₂N).
MH⁺ = 793.

**Exemple 3 : Synthèse du composé 3** (N-ditétradécylcarbamoylméthyl-2-{3-[4-(3-guanidino-propylamino)-butylamino]-propylamino}-acétamide) à partir du composé RPR 122766 (dont la préparation a été décrite dans la demande de brevet WO 97/18185 incorporée à la présente par référence).
0,784 mmol de RPR 122766 sont dissoutes dans 25 ml de méthanol dans un ballon équipé d'un barreau magnétique. On ajoute à cette solution 10,21 mmol de triéthylamine TEA. Puis, on coule lentement (5 minutes) sur le mélange 1,173 mmol d'une solution de O-méthylisourée/acide sulfurique H₂SO₄ dans 9 ml d'eau. En fin de coulée, un trouble apparaît. Le mélange est maintenu à 40°C pendant 16 heures, puis la solution est évaporée à sec. Le produit obtenu est ensuite purifié par HPLC préparative. Les fractions intéressantes sont regroupées et lyophilisées.
On obtient ainsi 0,2289 mmol de composé 3, soit un rendement de 29%.
HPLC analytique : Rt= 9,8 min.
Spectre de R M N ¹ H (400 MHz, (CD₃)₂SO d6, δ en ppm) : 0,87 (t, J = 7 Hz, 6H : CH₃ des chaînes grasses) ; de 1,15 à 1,40 (mt, 44H : (CH₂)₁₁ centraux des chaînes grasses) ; 1,46 et 1,55 (2 mts, 2H chacun : 1 CH₂ de chaque chaîne grasse) ; 1,63 (mt, 4H : les 2 CH₂ centraux du butyle) ; 1,81 et 1,95 (2 mts, 2H chacun : CH₂ central des propyles) ; de 2,85 à 3,10 (mt, 10H : les 2 NCH₂ du butyle - les 2 NCH₂ d' un des 2 propyles - et 1 des 2 NCH₂ de l'autre propyle) ; de 3,15 à 3,25 (mt, 6H : l'autre NCH₂ de l'autre propyle et NCH₂ des chaînes grasses) ; 3,82 (mf, 2H : NCH₂CON) ; 4,04 (d, J = 5 Hz, 2H : CONCH₂CON du glycyle) ; de 7,00 à 7,60 - de 8,60 à 8,75 et 9,00 (respectivement mf étalé et 2 mfs, 3H - 5H et 2H : NH₃⁺ CF₃COO⁻ - NH₂⁺ CF₃COO⁻ et =NH) ; 7,78 (t large, J = 5,5 Hz, 1H : N=CNH) ; 8,65 (mf : 1H correspondant au CONH).
MH⁺ = 751.

**Exemple 4 : Synthèse du composé** 4 (2-{2-[bis-(2-guanidino-éthyl)-amino]-éthylamino}-N-ditétradécylcarbamoylméthyl-acétamide)
Etape A : 10 excès molaires de tris(aminoéthyl)amine sont dissous dans 50 ml de dichlorométhane, et la solution est introduite dans le réacteur contenant une resine bromoacétyl(chloro)trityle (obtenue au préalable par réaction de l'acide bromoacétique sur une résine chlorotrityle). Le réacteur est agité pendant 2 heures à température ambiante. le solvant est filtré et la résine est lavée avec 10 fois 50 ml de dichlorométhane et d'isopropanol. Le test de Kaiser est positif.
Etape B : Les résines obtenues sont mises en contact avec un excès de 10 équivalents de 1,3-bis-(tert-butoxycarbonyl)-2-méthyl-é-thiopseudourée dissout dans du dichlorométhane, sous agitation, pendant 24 heures. le solvant est filtré et la résine est lavée avec 10 fois 50 ml de dichlorométhane et d'isopropanol. Le test de Kaiser à froid de 3 minutes est négatif.
Etape C : 50 mmol de DIEA sont dissoutes dans 50 ml de dichlorométhane, et le mélange est introduit dans le réacteur contenant le produit obtenu à l'étape B. Puis, on coule 48 mmol de dicarbonate de di-tert-butyle. Le réacteur est agité pendant une nuit. Le lendemain, le test de Kaiser est négatif. Le solvant est filtré, et la résine est lavée alternativement avec 10 fois 50 ml de dichlorométhane et d'isopropanol, 2 fois 50 ml deméthanol, et 2 fois 50 ml d'éther. la résine est ensuite séchée sous un courant d'azote. Le test de Kaiser est toujours négatif.
Etape D : Les résines obtenues à l'étape C sont introduites dans un ballon de 250 ml équipé d'un barreau magnétique. On y ajoute une solution composée de 50 ml de dichlorométhane et de 25 ml de 1,1,1,-trifluoroéthan-2-ol, et le mélange est agité pendant 2 heures. La solution est filtrée, et la résine est lavée avec 2 fois 10 ml de dichlorométhane. Les phases organiques ainsi obtenues sont rassemblées et évaporées sous vide. Les produits sont ensuite purifié sur gel de silice (éluant : chloroforme/méthanol 8:2 en volume). Les fractions intéressantes sont combinées puis évaporées sous vide pour obtenir 168 mg de produit de structure suivante : 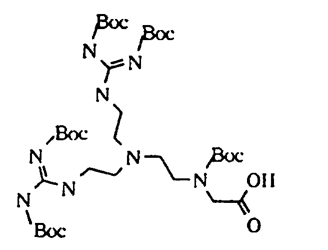 soit un rendement de 20%.
CCM : Rf = 0,9.
MH⁺ = 789.
Etape E : 9 mmol de Boc-glycinyl-di-tétradécylamide sont introduites dans un ballon équipé d'un barreau magnétique. 30 ml d'acide trifluoroacétique à 4°C sont ajoutés. La solution est agitée pendant une heure, et le TFA est évaporé sous vide. Le produit obtenu est à nouveau dissous par addition de 70 ml de DMF, puis on ajoute 30 mmol de TEA et 9 mmol de l'acide obtenu précédemment. Le pH est ajusté à 10, et 33 mmol de BOP sont ajoutées. La solution est agitée pendant 2 heures et suivie par CCM. Lorsque le couplage est achevé, 700 ml d'une solution de sulfate de potassium sont ajoutés et le produit est extrait avec 3 fois 100 ml d'acétate d'éthyle. La phase organique est lavée avec 3 fois 50 ml de sulfate de potassium, 3 fois 50 ml d carbonate de sodium, et 3 fois 50 ml de chlorure de sodium. Puis elle est séchée sur sulfate de magnésium, filtrée et évaporée sous vide. Le produit obtenu est analysé par RMN, CCM et MS, et est déprotégé avec 50 ml de TFA qui sont ajoutés au produit obtenu sans purification préalable. La solution est ensuite agitée pendant 1 heure et demi. Enfin, le TFA est évaporé et les produits finaux sont purifiés par CLHP semipréparative.
On obtient finalement 8,1 mmol de composé 4, soit un rendement de 90% pour cette dernière étape.
HPLC analytique : Rt = 11,4 min.
RMN ¹H (400 MHz, (CD₃)₂SO d6, δ en ppm) ; 0,89 (t, J = 7 Hz, 6H : CH₃ des chaînes grasses) ; de 1,15 à 1,35 (mt, 44H : (CH₂)₁₁ centraux des chaînes grasses) ; 1,45 et 1,54 (2 mts, 2H chacun : 1 CH₂ de chaque chaîne grasse) ; 2,65 - 2,78 - 3,06 et 3,23 (respectivement t, J = 6,5 Hz - t large, J = 6,5 Hz -mf et mt, respectivement 4H - 2H - 2H et 8H : NCH₂CH₂N - les 2 NCH₂CH₂NC=N et les NCH₂ des chaînes grasses) ; 3,83 (s large, 2H : NCH₂CON) ; 4,04 (d, J = 5 Hz, 2H : CONCH₂CON du glycyle) ; 7,34 (mf étalé, =NH et NH₂) ; 7.61 (t, J = 5.5 Hz, 2H : NH) ; 8,65 (t, J = 5 Hz, 1H : NH) ; 8,75 (mf : NH).
MH⁺ = 737.
La glycinyl-di-tétradécylamide utilisée dans l'étape E est préalablement obtenue de la façon suivante :
10 mmol de glycine protégée par des substituants Boc et 10 mmol de di-tétradécylamine sont introduites sans un ballon de250 ml. On y ajoute 100 ml de chloroforme, et le mélange est agité jusqu'à dissolution complète. On ajoute ensuite 30 mmol de TEA et 33 mmol de BOP. Le pH est maintenu à 10 grâce à la triéthylamine. La réaction est agitée pendant 2 heures. Lorsque la réaction, suivie par CCM, est achevée, le chloroforme est évaporé. On obtient un solide qui est à nouveau dissout dans 300 ml de d'acétate d'éthyle. la phase organique est ensuite lavée avec 4 fois 100 ml de sulfate de potassium, 4 fois 100 ml de carbonate de sodium, et 4 fois 100 ml de chlorure de sodium. Puis la phase organique est séchée sur sulfate de magnésium, filtrée et évaporée sous vide. Le produit obtenu est analysé par CCM, RMN et MS, et est utilisé sans autres purifications.

**Exemple 5 : Synthèse du composé 5** (N-ditétradécylcarbamoylméthyl-2-{(3--guanidino-propyl)-[4-(3-guanidino-propylamino)-butyl]-amino}-acétamide)
On opère de la même façon que précédemment pour le composé 4, mais à partir de spermine comme polyamine de départ, pour obtenir l'acide de structure suivante : 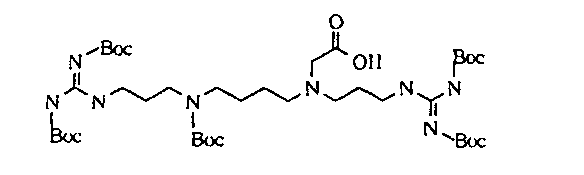 Après avoir été clivé de la resine, ce résidu est purifié sur gel de silice (éluant : chloroforme/méthanol 8:2 en volume). Les fractions intéressantes sont mélangées puis évaporées sous vide pour obtenir 0,69 mmol de la dite molécule, soit un rendement de 50%.
CCM : Rf = 0,5.
MH⁺ = 845.

Le composé 5 est obtenu par couplage entre l'acide précédemment synthétisé et la glycinyl-di tétradécylamide selon la même méthode que celle décrite à l'exemple 4. La glycinyl-di tétradécylamide est également obtenue de la même façon que précédemment.
On obtient ainsi 0,65 mmol de composé 5, soit un rendement de 94%pour cette étape.
HPLC analytique : Rt = 10,1 min.
Spectre de RMN ¹H (400 MHz, (CD₃)₂SO d6, à une température de 393 K, δ en ppm) : 0,92 (t, J = 7 Hz, 6H : CH₃ des chaînes grasses) ; de 1,25 à 1,45 (mt, 44H : (CH₂)₁₁ centraux des chaînes grasses) ; 1.57 (mt, 4H : les 2 CH₂ centraux du butyle) ; 1,57 et 1,67 (2 mts, 2H chacun : 1 CH₂ de chaque chaîne grasse) ; 1.74 et 1,91 (2 mts, 2H chacun: CH₂ central des propyles) ; 2,62 - 2,85 - de 3,20 à 3,35 (3 mts, 16H en toptalité : les NCH₂ - les CH₂NC=N et les NCH₂ des chaînes grasses) ; 3.17 (s, 2H : NCH₂CON) ; 4.02 (d, J = 5 Hz, 2H : CONCH₂CON du glycyle) ; 6.89 - de 7,30 à 7,55 et 7,65 (respectivement mf - mf étalé et mf : les H échangeables).
MH⁺ = 845.

**Exemple 6 : Synthèse du composé 6** (2-{3-[{4-[bis-(3-guanidino-propyl)-amino]-butyl}-(3-guanidino-propyl)-amino]-propylamino}-N-ditétradécylcarbamoylméthylacétamide)
On opère de la même façon que précédemment pour les composés 4 et 5, mais à partir de la N,N-N',N'(tétraaminopropyl)butylène diamine comme polyamine de départ, pour obtenir l'acide de structure suivante : 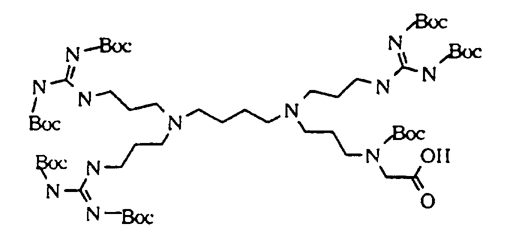 Après avoir été clivé de la résine, le résidu obtenu est purifié sur gel de silice (éluant : chloroforme/méthanol 8:2 en volume). Les fractions intéressantes sont mélangées puis évaporées sous vide pour obtenir 0,099 mmol de produit, soit un rendement de 20%.
CCM : Rf = 0,3.
MH⁺ = 1201.
Le composé 6 est obtenu par couplage entre l'acide précédemment synthétisé et la glycinyl-di tétradécylamide selon la même méthode que celle décrite à l'exemple 4. La glycinyl-di tétradécylamide est également obtenue de la même façon que précédemment.
On obtient ainsi 0,09 mmol de composé 6, soit un rendement de 90% pour cette étape.
HPLC analytique : Rt = 11,2 min.
Spectre de RMN ¹H (400 MHz, (CD₃)₂SO d6 avec ajout de quelques gouttes de CD₃COOD d4, δ en ppm) : 0,87 (t, J = 7 Hz, 6H : CH₃ des chaînes grasses) ; de 1,20 à 1,40 (mt, 44H : (CH₂)₁₁ centraux des chaînes grasses) ; 1,45 et 1,54 (2 mts, 2H chacun : 1 CH₂ de chaque chaîne grasse) ; 1,67 (mt, 4H : CH₂ centraux du butyle) ; de 1,80 à 2,10 (mt, 8H : CH₂ central des propyles) ; de 2,95 à 3,30 (mt, 20H : les NCH₂ des propyles et les NCH₂ du butyle) ; de 3.15 à 3,30 (mt, 4H : les NCH₂ des chaînes grasses) ; 3,84 (s, 2H : NCH₂CON) ; 4,05 (s, 2H : CONCH₂CON du glycyle).
MH⁺ = 949.

**Exemple 7 : utilisation du composé 1 pour la transfection *in vitro* de matériel génétique**

### Matériel génétique utilisé :

L'acide nucléique utilisé est le plasmide pXL2774 (WO97/10343) comportant le gène codant pour la luciférase sous le contrôle du promoteur du cytomegalovirus humain (CMV).

Les solutions d'acide nucléique sont diluées à 20 µg/ml dans du sérum physiologique (chlorure de sodium NaCl 0,15 M).

### Solutions de transfection (préparation extemporanée)

Les produits décrits dans l'invention sont mis en solution dans l'eau à concentration variant de 60 à 240 µM et mélangés volume à volume avec la solution d'ADN. La concentration satine finale est de 75 mM.

### Transfection

Les cellules sont cultivées dans les conditions appropriées en microplaques de 24 puits (2 cm²/puits), et sont transfectées alors qu'elles sont en phase exponentielle de croissance et à 50-70 % de la confluence.

Les cellules sont lavées par 2 fois 500 µl de milieu dépourvu de protéines sériques et remises en croissance soit en milieu sans sérum [transfection en l'absence de sérum], soit en milieu complet [transfection en présence de sérum]. 50 µl de mélange transfectant [soit 0,5µg d'ADN/puits] sont ajoutés aux cellules [3 puits/condition vecteur-DNA]. Quand les cellules sont transfectées en l'absence de sérum, le milieu de croissance est supplémenté par la quantité appropriée de sérum 2 heures après la transfection.

L'efficacité de transfection est évaluée 48 heures après la transfection par une mesure de l'expression de la luciférase selon les recommandations données pour l'utilisation du kit Promega [Luciferase Assay System]. La toxicité des mélanges transfectants est estimée par une mesure des concentrations protéiques dans les lysats cellulaires.

### Résultats

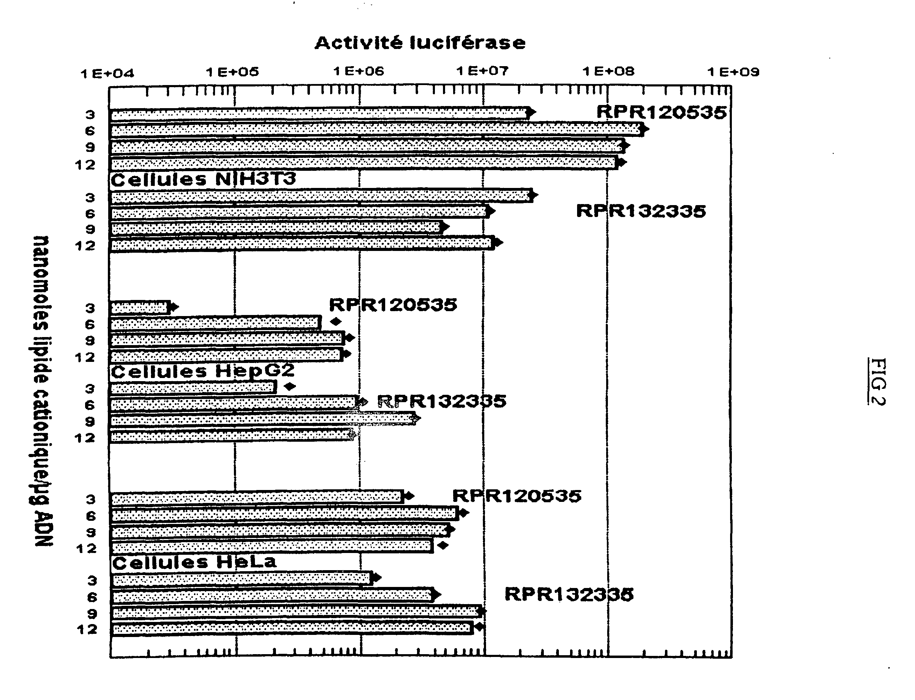

Le composé 1 a été utilisé comparativement au lipide cationique RPR 120535 (sel de TFA) [décrit dans la demande de brevet WO 97/18185 incorporée à la présente par référence] comme vecteur d'ADN pour transfecter trois lignées cellulaires différentes : les cellules NIH3T3, les cellules HepG2 et les cellules HeLa. Pour ces trois types cellulaires nous n'avons pas mis en évidence de toxicité significative du composé 1 dont la préparation est décrite dans l'exemple 1. L'efficacité de transfection du composé 1 et du lipide cationique de comparaison dans les trois types cellulaires est représentée à la figure 2/15, pour des rapports nmol de composé/µg d'ADN compris entre 3 et 12.

De la figure 2/15, on peut déduire que l'efficacité maximale de transfection est obtenue pour un ratio de 6 nanomoles lipide cationique/µg d'ADN pour les cellules NIH3T3 et pour un ratio de 9 nanomoles lipide cationique/µg d'ADN pour les cellules HeLa ou HepG2. Pour une transfection en l'absence de sérum, les niveaux d'expression obtenus dans les cellules HeLa ou HepG2 sont de 2 à 4 fois plus élevés pour le composé 1 que pour le produit testé en comparaison.

La figure 3/15 représente l'efficacité de transfection des complexes composé 1/ADN comparativement au même lipide cationique de référence que précédemment (RPR120535) complexé à l'ADN. Les barres blanches représentent l'efficacité de transfection des complexes nucléolipidiques en l'absence de sérum pendant 2 heures. Les barres noires représentent l'efficacité de transfection des complexes nucléolipidiques en présence de sérum.

De la figure 3/15 on peut ainsi mettre en évidence un des avantages particuliers des agents de transfert selon l'invention. En effet, on observe que les niveaux de transfection avec ou sans protéines sériques sont les mêmes dans le cas du composé 1 de l'invention, alors qu'avec le lipide cationique de comparaison, on observe une forte inhibition due à la présence des protéines sériques. Ceci constitue une propriété particulièrement intéressante pour des transfections *in vivo*.

### Exemple 8 : utilisation des composés 2, 3, 5 et 6 pour la transfection in vitro de matériel génétique

### Matériel génétique utilisé :

L'acide nucléique utilisé est le plasmide pXL3031 comportant le gène codant pour la luciférase sous le contrôle du promoteur du cytomegalovirus humain (CMV). Ce plasmidepXL3031 est représenté à la figure 15/15. Il a été isolé selon des protocoles standards connus de l'homme du métier, et notamment selon Maniatis T., Fritsch E.F. and Sambrook J., *Methods in Molecular Biology : a Laboratory Manual,* 1982, pp. 83-94, Cold Spring Harbor Lab., NY.
Plus concrètement, les méthodes utilisées sont la méthode de lyse alcaline et la purification sur gradient de chlorure de césium.

### Transfection :

Les cellules sont cultivées dans les conditions appropriées en microplaques de 24 puits. Après croissance pendant une nuit, chaque puit contient environ 100 000 cellules.

Dans chaque puit, on introduit le mélange transfectant qui contient 1 µg d'ADN dans 0,5 ml de DMEM, en l'absence de sérum. 5 heures après la transfection, le milieu de croissance est supplémenté par la quantité appropriée de sérum (DMEM et sérum de veau foetal, 10%). Les lysats cellulaires sont récupérés 24 heures après la transfection, et l'efficacité de la transfection est évaluée par la mesure de l'expression de la luciférase selon les recommandations données pour l'utilisation du kit Promega (Luciferase Detection Kit).

### Résultats :

Les niveau de transfection sont représentés aux figures 4/15, 5/15, 6/15 et 7/15.

A chaque fois, l'efficacité de transfection a été mesurée pour le lipide sous forme de micelles, et en mélange avec un co-lipide (DOPE ou bien cholestérol). De ces tableaux, on peut déduire que les niveaux de transfection sont très élevés, même à des rapports de charge très bas, avec les conséquences bénéfiques qui en résultent sur la toxicité. Ceci constitue l'un des avantages des composés selon l'invention. En effet, avec les lipides cationiques ne contenant pas de fonction(s) amidine, il est souvent nécessaire de se placer à des rapports de charge très élevés pour obtenir de tels niveaux de transfection, ce qui entraine en général une toxicité accrue.

### Exemple 9 : étude de l'affinité des composés 2, 3, 5 et 6 pour l'ADN

L'acide nucléique utilisé est le plasmide pXL3031, comme décrit à l'exemple précédent.

Les complexes sont préparés à des concentrations de 0,25 mg d'ADN/ml avec une quantité appropriée de lipide tel que défini dans la présente invention selon le rapport de charge souhaité. Le complexe est préparé dans un milieu contenant 5% de glucose et une solution de chlorure de sodium 20 mM. 50 µl de complexe nucléolipidique sont ensuite dilués 20 fois dans 950 µl de solution contenant 5% de glucose et une solution de chlorure de sodium 20mM.

La taille des complexes a été déterminée en mesurant le diamètre hydrodynamique par diffusion dynamique de la lumière à l'aide d'un appareil Coulter N4+.

Pour tous les composés, 3 phases physico-chimiques distinctes ont été mises en évidence selon le rapport de charge :
- La phase A pour laquelle l'ADN n'est pas saturé par le lipide cationique, c'est-à-dire qu'il reste encore de l'ADN nu dans le mélange, ce qui signifie que l'ADN n'est pas totalement protégé par le lipide et peut donc être soumis aux dégradations par les enzymes. Les complexes formés sont chargés globalement négativement, ce qui rend le passage des membranes cellulaires difficile. Il est ainsi préférable de ne pas se situer dans cette zone pour transfecter de l'ADN.
- La phase B pour laquelle l'ADN est complètement saturé par le lipide cationique et les complexes sont globalement neutres ou légèrement positifs. Les répulsions ioniques étant maximales, cette phase est instable. Un phénomène de "cross-linking" paut se produire conduisant à la précipitation des complexes. Les complexes dans cet état ne peuvent donc pas être utilisés en injection.
- La phase C pour laquelle l'ADN est sur-saturé par le lipide et les complexes sont donc globalement positifs. L'ADN est donc complètement protégé par le lipide et son passage à travers la membrane cellulaire (globalement négative) est facilitée. Les complexes de la phase C sont donc particulièrement adaptés à une utilisation pour le transfert d'acides nucléiques dans les cellules.

Les tableaux indiquant la phase dans laquelle les complexes se situent en fonction du rapport de charge sont représentés aux figures 8/15, 9/15, 10/15 et 11/15.

Les tableaux des figures 8/15, 9/15, 10/15 et 11/15 comparent les phases en fonctions des rapports de charge entre les composés de l'invention et leurs analogues aminés, c'est-à-dire les lipides cationiques dont la fonction amidine ou guanidine est remplacée par une fonction amino. On constate que la phase B est déplacée vers des rapports de charge beaucoup plus faibles. Ainsi, le fait d'incorporer des fonctions amidines dans la tête cationique contribue à l'augmentation de l'affinité des composés pour l'ADN. Ceci constitue une propriété importante des composés de l'invention car les complexes qu'ils forment avec l'ADN peuvent être utilisés dans la phase C sans pour autant se situer à des rapports de charge trop élevés, avec les effets bénéfiques qui en résultent sur la toxicité.

L'affinité des composés de l'invention pour l'ADN a également été évaluée à l'aide de la mesure de la réduction de fluorescence après ajout de bromure d'éthidium. En effet, la substitution du bromure d'éthidium de l'ADN par le lipide est une indication de liaison à l'ADN.

Ainsi, on ajoute aux complexes préparés 4 µl d'une solution de bromure d'éthidium 1 mg/ml, puis on mesure la fluorescence à une longueur d'onde d'excitation de 260 nm et à une longueur d'onde d'émission de 590 nm. La fluorescence obtenue pour l'ADN seul est défini comme étant de 100%.

Les résultats sont indiqués dans les tableaux des figures 12/15, 13/15 et 14/15. Ces résultats indiquent que les composés de l'invention présentent une très bonne affinité pour l'ADN, ce qui constitue une propriété particulièrement intéressante des composés de l'invention.

## Revendications

1. Lipopolyamine, sous forme D, L ou DL, ainsi que ses sels, de formule générale (I): 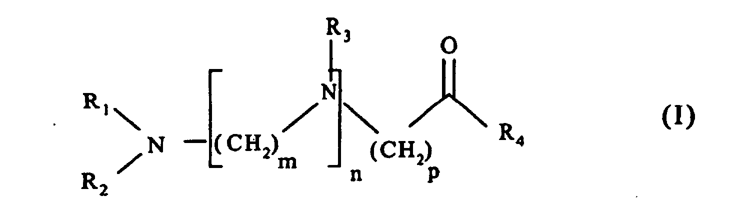 dans laquelle :
- R₁, R₂ et R₃ représentent indépendamment l'un de l'autre un atome d'hydrogène ou un groupement -(CH₂)_{q}-NRR' avec
• q étant un entier de 1 à 6 inclus, les valeurs de q étant indépendantes les une des autres entre les différents groupements R₁, R₂ et R₃, et
• R et R' représentent indépendamment l'un de l'autre un atome d'hydrogène ou un groupement de formule (II) : 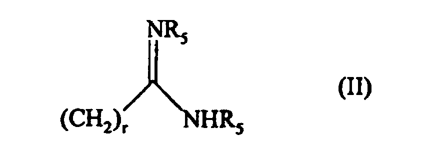
dans laquelle r est un nombre entier pouvant varier de 0 à 6 inclus, et les groupements R₅ représentent, indépendamment l'un de l'autre, un atome d'hydrogène ou un substituant alkyle, carbamate ou acyle aliphatique ou aromatique, éventuellement halogéné,
étant entendu que l'un au moins des groupes R₁, R₂ et R₃ comporte au moins un groupement de formule (II),
- m et n représentent, indépendamment l'un de l'autre, un nombre entier pouvant varier de 1 à 6 inclus avec, lorsque n est supérieur à 1, m pouvant prendre des valeurs différentes et R₃ des significations différentes au sein de la formule générale (I),
- p représente un nombre entier pouvant varier de 1 à 6 inclus, et,
- R₄ représente un groupement de formule générale (III) : 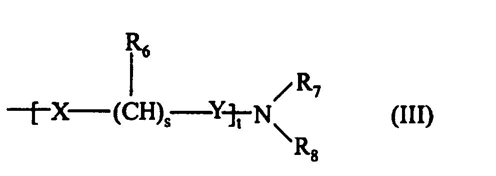
dans laquelle:
• R₇ et R₈ représentent indépendamment l'un de l'autre un atome d'hydrogène, ou un radical aliphatique contenant 5 à 22 atomes de carbone, saturé ou non, linéaire ou ramifié, éventuellement halogéné, ou un dérivé de stéroïde, ou un groupement (CH₂)ᵤ-NH-R₉ dans lequel u est un nombre entier compris entre 2 et 6 inclus et R₉ est un radical acyle, l'un au moins des groupes R₇ et R₈ étant différent de l'hydrogène,
• t est un nombre entier choisi de 0 à 10 inclus avec R₆, X, Y et s pouvant avoir des significations différentes au sein des différents motifs [X-(CHR₆)ₛ-Y] lorsque t est un entier supérieur à 1,
• X représente un atome d'oxygène ou de soufre, ou un groupement amino ou alkylamino, le substituant alkyle étant linéaire ou ramifié et contenant 1 à 8 atomes de carbone,
• Y représente un groupement carbonyle ou un groupement méthylène,
• R₆ représente un atome d'hydrogène ou une chaîne latérale d'acide aminé naturel, le cas échéant substituée, et,
• s représente un entier variant de 1 à 10 inclus avec lorsque s est égal à 1, R₆ représentant une chaîne latérale d'acide aminé naturel, le cas échéant substituée, et lorsque s est supérieur à 1, R₆ représentant un atome d'hydrogène.

2. Composés selon la revendication 1 **caractérisés en ce que**, dans la formule (II), l'un des groupes R₅ représente un atome d'hydrogène et l'autre un reste aliphatique contenant 1 à 10 atomes de carbone ou un reste aromatique.

3. Composés selon la revendication 1 **caractérisés en ce que**, dans la formule (II), l'un des groupes R₅ représente un atome d'hydrogène et l'autre le benzyle ou l'un de ses dérivés.

4. Composés selon la revendication 1 **caractérisés en ce que**, dans la formule (II), les deux groupes R₅ représentent des atomes d'hydrogène.

5. Composés selon l'une des revendications précédentes **caractérisés en ce que**, lorsque R₁, R₂, et/ou R₃ sont différents de l'hydrogène et comprennent la formule (II), q prend la valeur 2 ou 3 et r vaut 0.

6. Composés selon l'une des revendications précédentes **caractérisés en ce que**, dans la formule générale (I), m est choisi parmi 2, 3 et 4.

7. Composés selon l'une des revendications précédentes caractérisés en ce l'un au moins des groupes R₇ et R₈ est représenté par un dérivé de stéroïde choisi parmi le cholestérol, le cholestanol, le 3-α-5-cyclo-5-α-cholestan-6-β-ol, l'acide cholique, le cholestéryl-formiate, le chotestanylformiate, le 3α,5-cyclo-5α-cholestan-6β-yl formiate, la cholestérylamine, la 6-(1,5-diméthylhexyl)-3a,5a-diméthylhexadécahydrocyclo-penta[a]cyclopropa[2,3]cyclopenta[1,2-f]naphta-lèn-10-ylamine, ou la cholestanyl-amine.

8. Composés selon l'une des revendications 1 à 6 **caractérisés en ce que** l'un au moins des groupes R₇ et R₈ est représenté par un groupement (CH2)ᵤ-NH-R₉ dans lequel u est un nombre entier compris entre 2 et 6 inclus et R₉ est un radical acyle.

9. Composés selon la revendication 8 **caractérisés en ce que** le groupement R₉ est le cholestérylformiate, l'arachidonyle ou l'acide cholique.

10. Composés selon la revendication 1 **caractérisés en ce que** les deux groupes R₇ et R₈ représentent indépendamment l'un de l'autre un radical aliphatique contenant 5 à 22 atomes de carbone, saturé ou non, linéaire ou ramifié, éventuellement halogéné, ou un dérivé de stéroïde, ou un groupement (CH₂)ᵤ-NH-R₉ dans lequel u est un nombre entier compris entre 2 et 6 inclus et R₉ est un radical acyle.

11. Composés selon la revendication 10 **caractérisés en ce que** les deux groupes R₇ et R₈ représentent une chaîne aliphatique contenant 5 à 22 atomes de carbone, et de préférence entre 12 et 22 atomes de carbone.

12. Composés selon la revendication 1 **caractérisés en ce que** R₁ comporte un groupement de formule (II) et R₂ et R₃ sont des atomes d'hydrogène.

13. Composés selon la revendication 1 **caractérisés en ce que** R₁ et R₂ comportent chacun un groupement de formule (II) et R₃ est un atome d'hydrogène.

14. Composés selon la revendication 1 **caractérisés en ce que** R₁ et R₃ comportent chacun un groupement de formule (II) et R₂ est un atome d'hydrogène.

15. Composés selon la revendication 1 **caractérisés en ce que** R₁, R₂ et R₃ comportent chacun un groupement de formule (II).

16. Composés selon l'une des revendications précédentes **caractérisés en ce qu'**il est associé à un élément de ciblage extra ou intracellulaire.

17. Composés selon la revendication 16 **caractérisés en ce qu'**il incorpore l'élément de ciblage au niveau de la chaîne latérale d'acide aminé R₆.

18. Composés selon l'une des revendications précédentes **caractérisés en ce qu'**il incorpore un agent marqueur de type biotine, rhodamine, folate, ou une séquence peptidique ou pseudopeptidique linéaire ou cyclique comportant l'épitope Arg-Gly-Asp au niveau de la chaîne latérale d'acide aminé R₆.

19. Composés selon la revendication 1 **caractérisés en ce qu'**il est choisi parmi les composés de formule suivantes dans lesquelles R₄ et R₅ ont les significations données dans la revendication 1 :

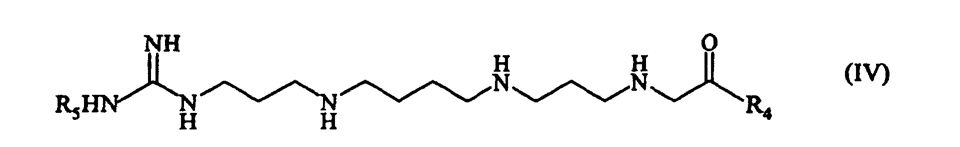

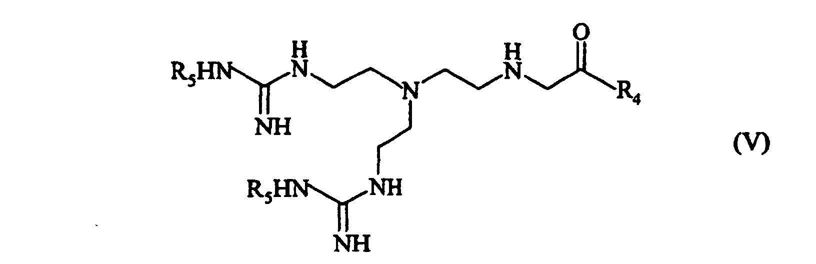

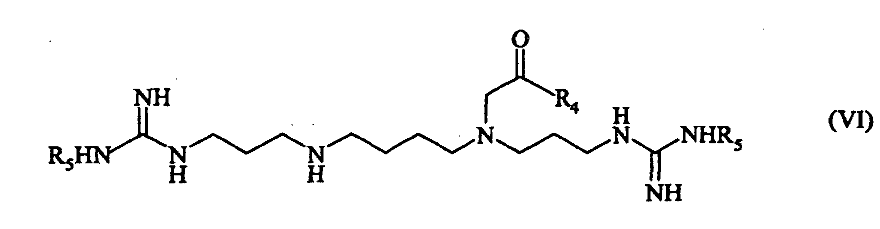

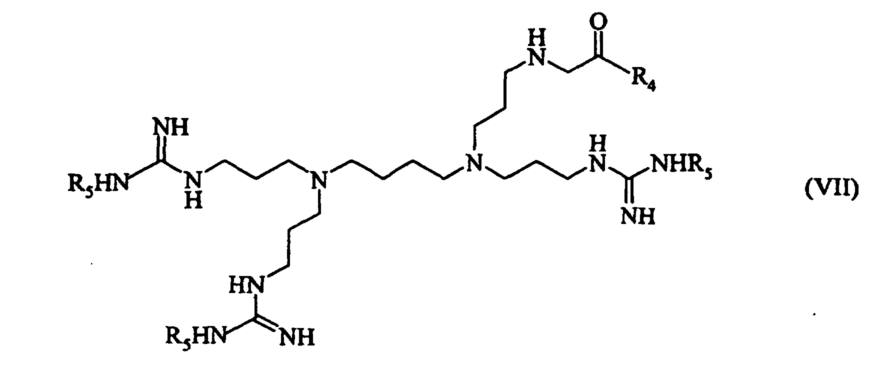

20. Composés selon la revendication 1 **caractérisés en ce qu'**il est choisi parmi

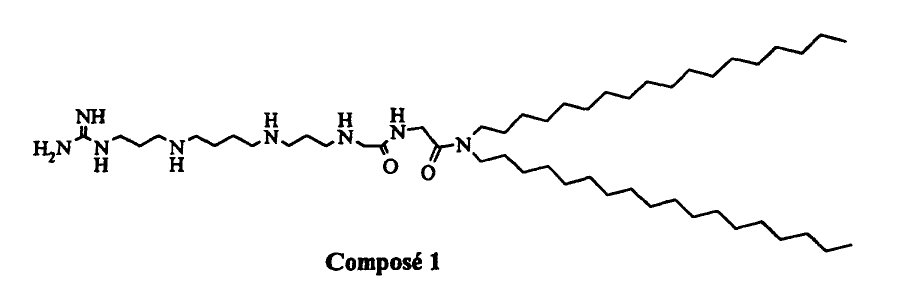

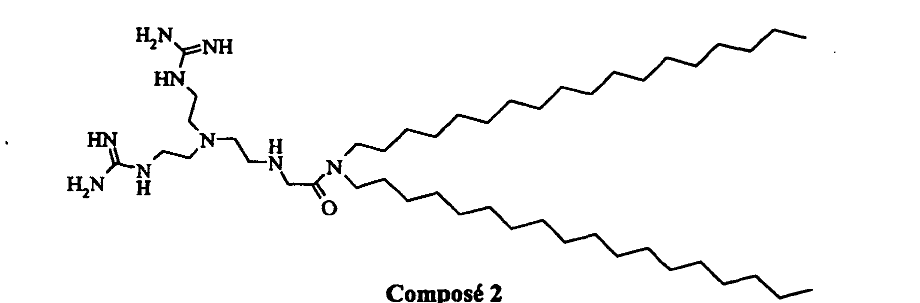

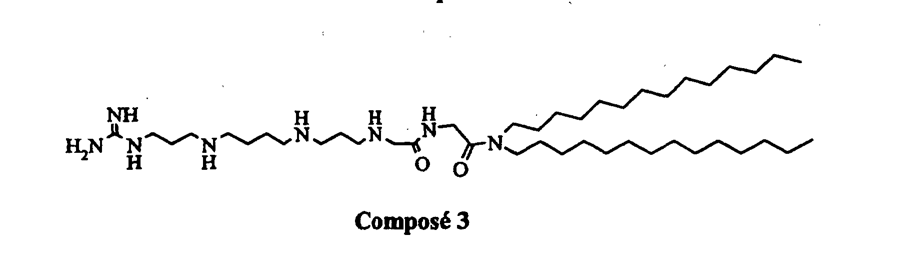

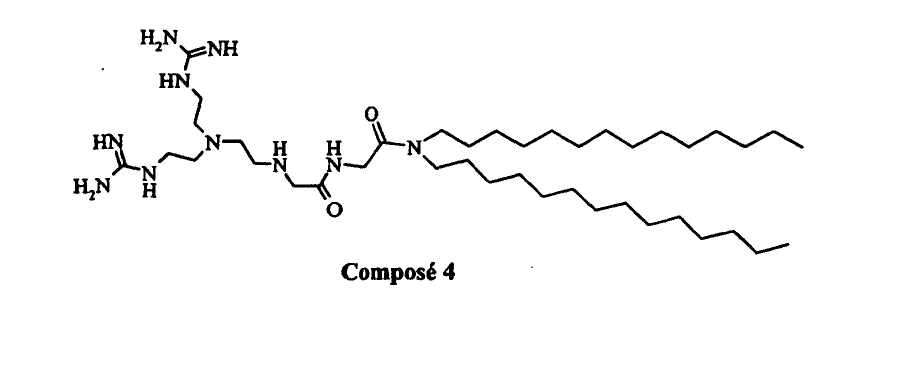

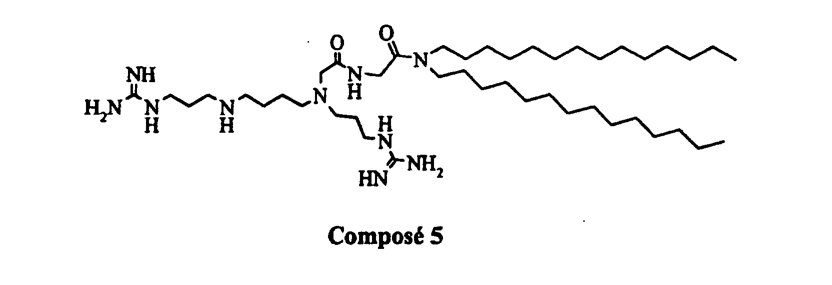

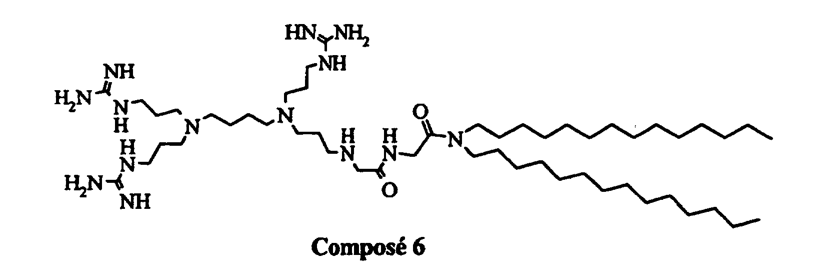

21. Composition comprenant un composé selon l'une des revendications précédentes et un acide nucléique.

22. Composition selon la revendication 21 **caractérisée en ce qu'**elle comprend en outre un ou plusieurs adjuvants.

23. Composition selon la revendication 22 **caractérisée en ce que** l'adjuvant est un ou plusieurs lipides neutres.

24. Composition selon les revendications 22 et 23 **caractérisée en ce que** les lipides neutres sont des lipides à deux chaînes grasses.

25. Composition selon les revendications 22 à 24 **caractérisée en ce que** les lipides neutres sont des lipides naturels ou synthétiques, zwitterioniques ou dépourvus de charge ionique dans les conditions physiologiques, choisis par exemple parmi la dioléoylphosphatidyléthanolamine (DOPE), l'oléoylpalmitoylphosphatidyléthanolamine (POPE), les di-stéaroyl-, palmitoyl-, mirystoylphosphatidyléthanolamines ainsi que leurs dérivés N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

26. Composition selon la revendication 22 **caractérisée en ce que** l'adjuvant est un composé intervenant ou non directement au niveau de la condensation de l'acide nucléique.

27. Composition selon les revendications 22 à 24 **caractérisée en ce que** ledit adjuvant dérive en tout ou en partie d'une protamine, d'une histone, ou d'une nucléoline et/ou de l'un de leurs dérivés, ou bien est constitué, en tout ou en partie, de motifs peptidiques (KTPKKAKKP) et/ou (ATPAKKAA), le nombre des motifs pouvant varier entre 2 et 10 et pouvant être répétés de manière continue ou non.

28. Composition selon la revendication 21 **caractérisée en ce qu'**elle comprend en outre un élément de ciblage.

29. Composition selon les revendications 21 à 28 **caractérisée en ce qu'**elle comprend un véhicule pharmaceutiquement acceptable pour une formulation injectable.

30. Composition selon les revendications 21 à 28 **caractérisée en ce qu'**elle comprend un véhicule pharmaceutiquement acceptable pour une application sur la peau et/ou les muqueuses.

31. Utilisation d'un composé selon l'une des revendications 1 à 20 pour le transfert d'acides nucléiques dans les cellules.

32. Procédé de préparation d'un composé de formule générale (I): 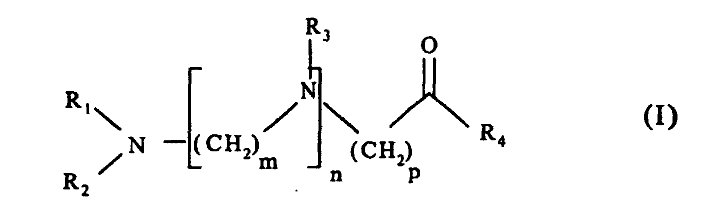 dans laquelle :
- R₁, R₂ et R₃ représentent indépendamment l'un de l'autre un atome d'hydrogène ou un groupement -(CH₂)_{q}-NRR' avec
• q étant un entier de 1 à 6 inclus, les valeurs de q étant indépendantes les une des autres entre les différents groupements R₁, R₂ et R₃, et
• R et R' représentent indépendamment l'un de l'autre un atome d'hydrogène ou un groupement de formule (II) : 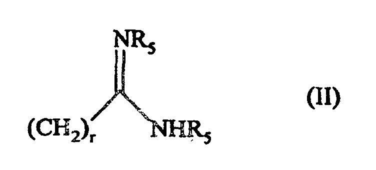
dans laquelle r est un nombre entier pouvant varier de 0 à 6 inclus, et les groupements R₅ représentent, indépendamment l'un de l'autre, un atome d'hydrogène ou un substituant alkyle, carbamate ou acyle aliphatique ou aromatique, éventuellement halogéné,
étant entendu que l'un au moins des groupes R₁, R₂ et R₃ comporte au moins un groupement de formule (II),
- m et n représentent, indépendamment l'un de l'autre, un nombre entier pouvant varier de 1 à 6 inclus avec, lorsque n est supérieur à 1, m pouvant prendre des valeurs différentes et R₃ des significations différentes au sein de la formule générale (I),
- p représente un nombre entier pouvant varier de 1 à 6 inclus, et,
- R₄ représente un groupement de formule générale (III) : 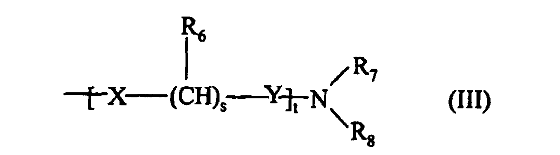
dans laquelle:
• R₇ et R₈ représentent indépendamment l'un de l'autre un atome d'hydrogène, ou un radical aliphatique contenant 5 à 22 atomes de carbone, saturé ou non, linéaire ou ramifié, éventuellement halogéné, ou un dérivé de stéroïde, ou un groupement (CH₂)ᵤ-NH-R₉ dans lequel u est un nombre entier compris entre 2 et 6 inclus et R₉ est un radical acyle, l'un au moins des groupes R₇ et R₈ étant différent de l'hydrogène,
• t est un nombre entier choisi de 0 à 10 inclus avec R₆, X, Y et s pouvant avoir des significations différentes au sein des différents motifs [X-(CHR₆)_{S}-Y] lorsque t est un entier supérieur à 1,
• X représente un atome d'oxygène ou de soufre, ou un groupement amino ou alkylamino, le substituant alkyle étant linéaire ou ramifié et contenant 1 à 8 atomes de carbone,
• Y représente un groupement carbonyle ou un groupement méthylène,
• R₆ représente un atome d'hydrogène ou une chaîne latérale d'acide aminé naturel, le cas échéant substituée, et,
• s représente un entier variant de 1 à 10 inclus avec lorsque s est égal à 1, R₆ représentant une chaîne latérale d'acide aminé naturel, le cas échéant substituée, et lorsque s est supérieur à 1, R₆ représentant un atome d'hydrogène,
**caractérisé en ce que** l'on forme un groupement amidine sur par action d'un dérivé thio ou oxo de l'urée sur une lipopolyamine ou **en ce que** l'on greffe une polyaminoamidine à un lipide par couplage peptidique.

33. Méthode de transfert d'acides nucléiques dans les cellules **caractérisée en ce qu'**elle comprend les étapes suivantes :
(1) la mise en contact de l'acide nucléique avec un composé tel que défini dans les revendications 1 à 20 et le cas échéant avec un ou plusieurs adjuvants et/ou élément de ciblage pour former un complexe et,
(2) la mise en contact des cellules avec le complexe formé en (1).

## Patentansprüche

1. Lipopolyamine in der Form D, L oder DL sowie ihre Salze, der allgemeinen Formel (I) 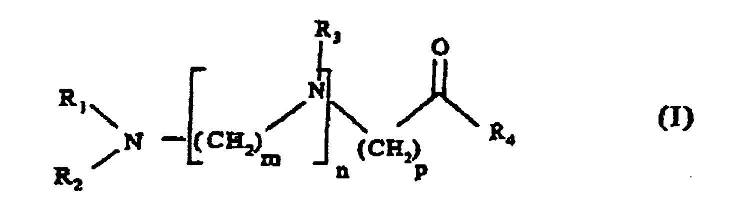 in der
R₁, R₂ und R₃, unabhängig voneinander, ein Wasserstoffatom oder eine Gruppe -(CH₂)_{q}-NRR' darstellen, mit
• q, das eine ganze Zahl von 1 bis einschließlich 6 darstellt, wobei die Werte von q unabhängig voneinander innerhalb der verschiedenen Gruppen von R₁, R₂ und R₃ sind, und
• R und R', unabhängig voneinander, ein Wasserstoffatom oder eine Gruppe der Formel (II) 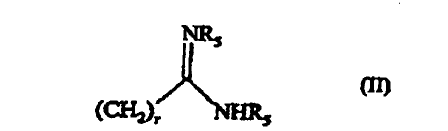 darstellen,
worin r eine ganze Zahl darstellt, die von 0 bis einschließlich 6 variieren kann, und die Gruppen R₅, unabhängig voneinander, ein Wasserstoffatom oder einen Substituenten Alkyl, Carbamat oder Acyl, aliphatisch oder aromatisch, gegebenenfalls halogeniert, darstellen,
mit der Maßgabe, daß mindestens eine der Gruppen R₁, R₂ und R₃ mindestens eine Gruppe der Formel (II) umfaßt,
- m und n, unabhängig voneinander, eine ganze Zahl darstellen, die von 1 bis einschließlich 6 variieren kann, wobei in dem Fall, wo n höher als 1 ist, m unterschiedliche Werte annehmen kann und R₃ die verschiedenen bei der allgemeinen Formel (I) angegebenen Bedeutungen besitzt,
- p eine ganze Zahl darstellt, die von 1 bis einschließlich 6 variieren kann, und
- R₄ eine Gruppe der allgemeinen Formel (III) 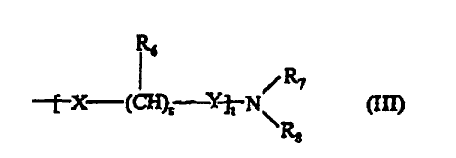 darstellt, in der
• R₇ und R₈, unabhängig voneinander, ein Wasserstoffatom oder einen linearen oder verzweigten, gesättigten oder ungesättigten aliphatischen Rest mit 5 bis 22 Kohlenstoffatomen, gegebenenfalls halogeniert, oder ein Steroid-Derivat oder eine Gruppe (CH₂)ᵤ-NH-R₉ darstellen, worin u eine ganze Zahl zwischen 2 und einschließlich 6 darstellt und R₉ ein Rest Acyl ist, wobei mindestens eine der Gruppen R₇ und R₈ von Wasserstoff verschieden ist,
• t eine ganze Zahl ist, ausgewählt von 0 bis einschließlich 10, wobei, wenn t eine ganze Zahl höher als 1 darstellt, R₆, X, Y und s innerhalb der verschiedenen Struktureinheiten [X-(CHR₆)ₛ-Y] verschiedene Bedeutungen besitzen können,
• X ein Atom von Sauerstoff oder Schwefel oder eine Gruppe Amino oder Alkylamino darstellt, worin der Substituent Alkyl linear oder verzweigt ist und 1 bis 8 Kohlenstoffatome enthält,
• Y eine Gruppe Carbonyl oder eine Gruppe Methylen darstellt,
• R₆ eine Wasserstoffatom oder eine Seitenkette einer natürlichen Aminosäure ist, gegebenenfalls substituiert, und
• s eine ganze Zahl darstellt, die von 1 bis einschließlich 10 variieren kann, wobei, wenn s gleich 1 ist, R₆ eine Seitenkette einer natürlichen Aminosäure darstellt, gegebenenfalls substituiert, und wenn s höher als 1 ist, R₆ ein Wasserstoffatom darstellt.

2. Verbindungen nach Anspruch 1, **dadurch gekennzeichnet, daß** in der Formel (II) eine der Gruppen R₅ ein Wasserstoffatom ist und die andere einen aliphatischen Rest mit 1 bis 10 Kohlenstoffatomen oder einen aromatischen Rest darstellt.

3. Verbindungen nach Anspruch 1, **dadurch gekennzeichnet, daß** in der Formel (II) eine der Gruppen R₅ ein Wasserstoffatom ist und die andere Benzyl oder eines seiner Derivate darstellt.

4. Verbindungen nach Anspruch 1, **dadurch gekennzeichnet, daß** in der Formel (II) beide Gruppen R₅ Wasserstoffatome darstellen.

5. Verbindungen nach einem der vorstehenden Ansprüche, **dadurch gekennzeichnet, daß** in dem Fall, wo R₁, R₂ und/oder R₃ von Wasserstoff verschieden sind und die Formel (II) umfassen, q den Wert 2 oder 3 annimmt und r 0 ist.

6. Verbindungen nach einem der vorstehenden Ansprüche, **dadurch gekennzeichnet, daß** in der allgemeinen Formel (I) m unter 2, 3 und 4 ausgewählt wird.

7. Verbindungen nach einem der vorstehenden Ansprüche, **dadurch gekennzeichnet, daß** mindestens eine der Gruppen R₇ und R₈ durch ein Steroid-Derivat dargestellt wird, ausgewählt unter Cholesterin, Cholestanol, 3α-5-Cyclo-5α-cholestan-6β-ol, Cholsäure, Cholesteryl-formiat, Cholestanyl-formiat, 3α-5-Cyclo-5α-cholestan-6β-yl-formiat, Cholesterylamin, 6-(1,5-Dimethylhexyl)-3a,5adimethylhexadecahydrocyclo-penta[a]-cyclopropa[2,3]-cyclopenta-[1,2-f]-naphthalin-10-ylamin oder Cholestanyl-amin.

8. Verbindungen nach einem der Ansprüche 1 bis 6, **dadurch gekennzeichnet, daß** mindestens eine der Gruppen R₇ und R₈ durch eine Gruppe (CH₂)ᵤ-NH-R₉ dargestellt wird, in der u eine ganze Zahl zwischen 2 und einschließlich 6 ist und R₉ einen Rest Acyl darstellt.

9. Verbindungen nach Anspruch 8, **dadurch gekennzeichnet, daß** die Gruppe R₉ Cholesteryl-formiat, Arachidonyl oder Cholsäure ist.

10. Verbindungen nach Anspruch 1, **dadurch gekennzeichnet, daß** die beiden Gruppen R₇ und R₈ unabhängig voneinander einen linearen oder verzweigten, gesättigten oder ungesättigten aliphatischen Rest mit 5 bis 22 Kohlenstoffatomen, gegebenenfalls halogeniert, oder ein Steroid-Derivat oder eine Gruppe (CH₂)ᵤ-NH-R₉ darstellen, worin u eine ganze Zahl zwischen 2 und einschließlich 6 darstellt und R₉ ein Rest Acyl ist.

11. Verbindungen nach Anspruch 10, **dadurch gekennzeichnet, daß** die beiden Gruppen R₇ und R₈ eine aliphatische Kette mit 5 bis 22 Kohlenstoffatomen, vorzugsweise zwischen 12 und 22 Kohlenstoffatomen darstellen.

12. Verbindungen nach Anspruch 1, **dadurch gekennzeichnet, daß** R₁ eine Gruppe der Formel (II) umfaßt und R₂ und R₃ Wasserstoffatome sind.

13. Verbindungen nach Anspruch 1, **dadurch gekennzeichnet, daß** R₁ und R₂ jeweils eine Gruppe der Formel (II) umfassen und R₃ ein Wasserstoffatom ist.

14. Verbindungen nach Anspruch 1, **dadurch gekennzeichnet, daß** R₁ und R₃ jeweils eine Gruppe der Formel (II) umfassen und R₂ ein Wasserstoffatom ist.

15. Verbindungen nach Anspruch 1, **dadurch gekennzeichnet, daß** R₁, R₂ und R₃ jeweils eine Gruppe der Formel (II) umfassen.

16. Verbindungen nach einem der vorstehenden Ansprüche, **dadurch gekennzeichnet, daß** sie mit einem extra- oder intrazellularen Ziel-Element assoziiert sind.

17. Verbindungen nach Anspruch 16, **dadurch gekennzeichnet, daß** sie das Ziel-Element im Bereich der Seitenkette der Aminosäure R₆ eingliedern.

18. Verbindungen nach einem der vorstehenden Ansprüche, **dadurch gekennzeichnet, daß** sie ein Marker-Mittel vom Typ Biotin, Rhodamin, Folat oder eine lineare oder cyclische, peptidische oder pseudopeptidische Sequenz eingliedern, die das Epitop Arg-Gly-Asp im Bereich der Seitenkette der Aminosäure R₆ umfaßt.

19. Verbindungen nach Anspruch 1, **dadurch gekennzeichnet, daß** sie unter den Verbindungen der folgenden Formeln ausgewählt werden, in denen R₄ und R₅ die in Anspruch 1 angegebenen Bedeutungen besitzen:

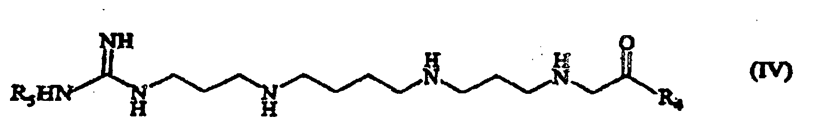

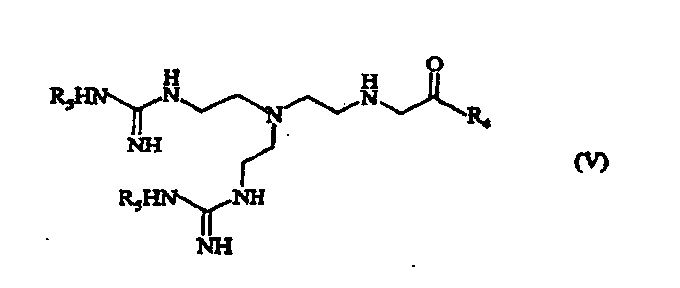

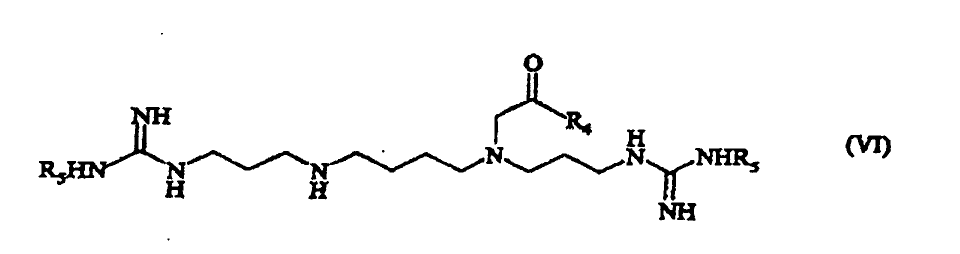

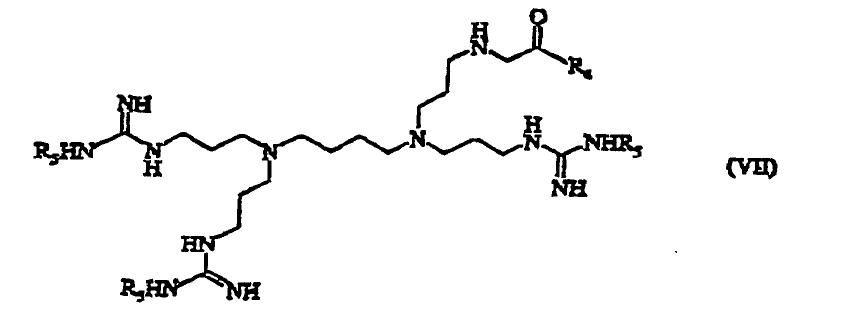

20. Verbindungen nach Anspruch 1, **dadurch gekennzeichnet, daß** sie ausgewählt werden unter

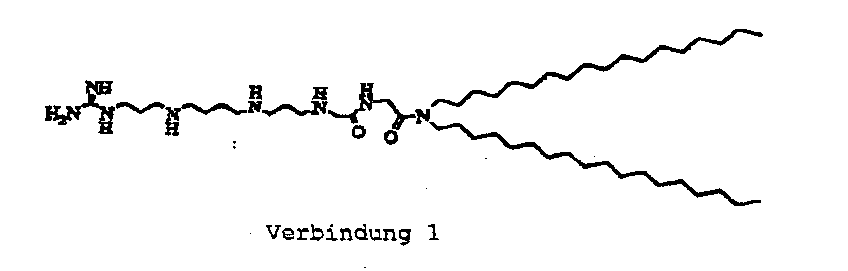

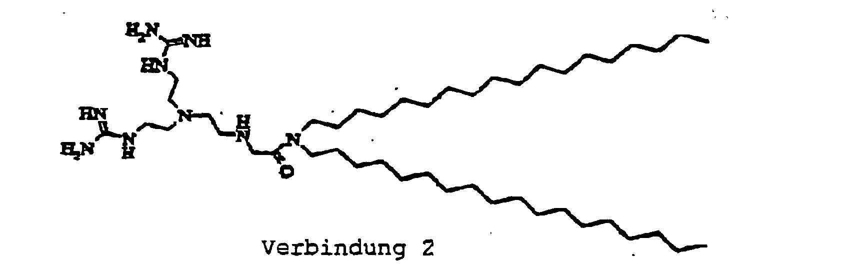

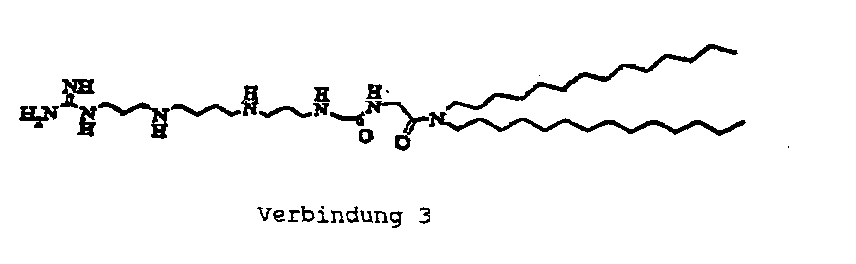

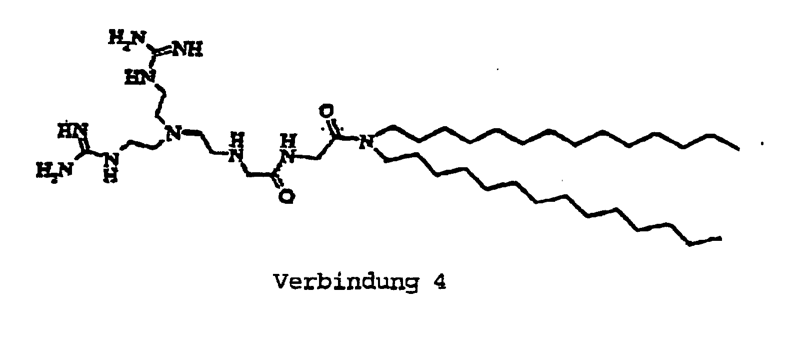

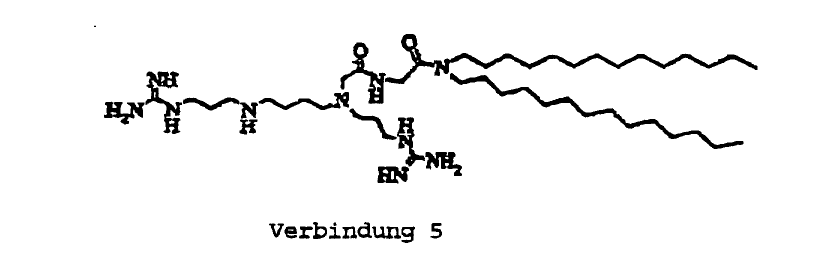

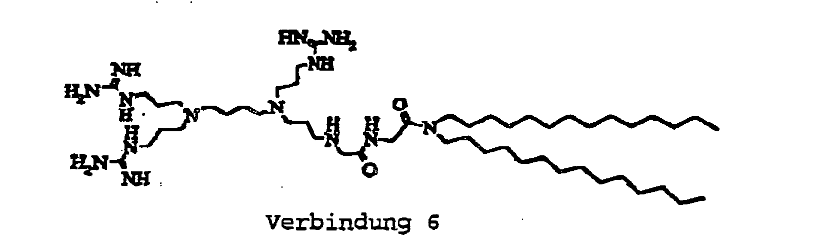

21. Zusammensetzung, umfassend eine Verbindung nach einem der vorstehenden Ansprüche und eine Nucleinsäure.

22. Zusammensetzung nach Anspruch 21, **dadurch gekennzeichnet, daß** sie außerdem einen oder mehrere Zusatzstoffe umfaßt.

23. Zusammensetzung nach Anspruch 22, **dadurch gekennzeichnet, daß** der Zusatzstoff aus einem oder mehreren neutralen Lipiden besteht.

24. Zusammensetzung nach Anspruch 22 und 23, **dadurch gekennzeichnet, daß** die neutralen Lipide Lipide mit zwei Fettketten sind.

25. Zusammensetzung nach Anspruch 22 bis 24, **dadurch gekennzeichnet, daß** die neutralen Lipide natürliche oder synthetische, zwitterionische oder unter physiologischen Bedingungen von ionischer Ladung freie Lipide sind, beispielsweise ausgewählt unter Dioleylphosphatidylethanolamin (DOPE), Oleylpalmitoylphosphatidylethanolamin (POPE), den Di-Stearoyl-, Palmitoyl-, Mirystoylphosphatidylethanolaminen sowie ihren ein- bis dreimal N-methylierten Derivaten, den Phosphatidylglycerinen, den Diacylglycerinen, den Glycosyldiacylglycerinen, den Cerebrosiden (wie insbesondere den Galactocerebrosiden), den Sphingolipiden (wie insbesondere den Sphingomyelinen) oder auch den Asialogangliosiden (wie insbesondere den asialoGM1 und GM2).

26. Zusammensetzung nach Anspruch 22, **dadurch gekennzeichnet, daß** der Zusatzstoff eine Verbindung ist, die im Bereich der Kondensation der Nucleinsäure eingreift oder nicht direkt eingreift.

27. Zusammensetzung nach Anspruch 22 bis 24, **dadurch gekennzeichnet, daß** sich der genannte Zusatzstoff ganz oder teilweise von einem Protamin, einem Histon oder einem Nucleolin und/oder einem seiner Derivate ableitet, oder auch ganz oder teilweise aus peptidischen Struktureinheiten (KTPKKAKKP) und/oder (ATPAKKAA) besteht, wobei die Anzahl der Struktureinheiten zwischen 2 und 10 variieren und sich in kontinuierlicher Weise oder auch nicht wiederholen kann.

28. Zusammensetzung nach Anspruch 21, **dadurch gekennzeichnet, daß** sie außerdem ein Ziel-Element umfaßt.

29. Zusammensetzung nach Anspruch 21 bis 28, **dadurch gekennzeichnet, daß** sie einen pharmazeutisch akzeptablen Trägerstoff für eine injizierbare Formulierung umfaßt.

30. Zusammensetzung nach Anspruch 21 bis 28, **dadurch gekennzeichnet, daß** sie einen pharmazeutisch akzeptablen Trägerstoff für eine Anwendung auf der Haut und/oder den Schleimhäuten umfaßt.

31. Verwendung einer Verbindung nach einem der Ansprüche 1 bis 20 für den Transfer von Nucleinsäuren in die Zellen.

32. Verfahren zur Herstellung einer Verbindung der allgemeinen Formel (I) 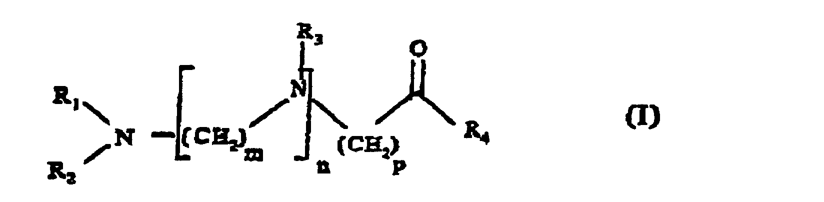 in der
R₁, R₂ und R₃, unabhängig voneinander, ein Wasserstoffatom oder eine Gruppe -(CH₂)_{q}-NRR' darstellen, mit
• q, das eine ganze Zahl von 1 bis einschließlich 6 darstellt, wobei die Werte von q unabhängig voneinander innerhalb der verschiedenen Gruppen R₁, R₂ und R₃ sind, und
• R und R', unabhängig voneinander, ein Wasserstoffatom oder eine Gruppe der Formel (II) 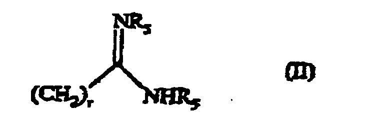 darstellen,
worin r eine ganze Zahl darstellt, die von 0 bis einschließlich 6 variieren kann, und die Gruppen R₅, unabhängig voneinander, ein Wasserstoffatom oder einen Substituenten Alkyl, Carbamat oder Acyl, aliphatisch oder aromatisch, gegebenenfalls halogeniert, darstellen,
mit der Maßgabe, daß mindestens eine der Gruppen R₁, R₂ und R₃ mindestens eine Gruppe der Formel (II) umfaßt,
- m und n, unabhängig voneinander, eine ganze Zahl darstellen, die von 1 bis einschließlich 6 variieren kann, wobei in dem Fall, wo n höher als 1 ist, m unterschiedliche Werte annehmen kann und R₃ die verschiedenen, bei der allgemeinen Formel (I) angegebenen Bedeutungen besitzt,
- p eine ganze Zahl darstellt, die von 1 bis einschließlich 6 variieren kann, und
- R₄ eine Gruppe der allgemeinen Formel (III) 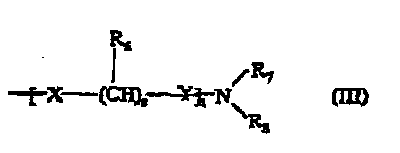 darstellt, in der
• R₇ und R₈, unabhängig voneinander, ein Wasserstoffatom oder einen linearen oder verzweigten, gesättigten oder ungesättigten aliphatischen Rest mit 5 bis 22 Kohlenstoffatomen, gegebenenfalls halogeniert, oder ein Steroid-Derivat oder eine Gruppe (CH₂)ᵤ-NH-R₉ darstellen, worin u eine ganze Zahl zwischen 2 und einschließlich 6 darstellt und R₉ ein Rest Acyl ist, wobei mindestens eine der Gruppen R₇ und R₈ von Wasserstoff verschieden ist,
• t eine ganze Zahl ist, ausgewählt von 0 bis einschließlich 10, wobei, wenn t eine ganze Zahl höher als 1 darstellt, R₆, X, Y und s innerhalb der verschiedenen Struktureinheiten [X-(CHR₆)ₛ-Y] verschiedene Bedeutungen besitzen können,
• X ein Atom von Sauerstoff oder Schwefel oder eine Gruppe Amino oder Alkylamino darstellt, worin der Substituent Alkyl linear oder verzweigt ist und 1 bis 8 Kohlenstoffatome enthält,
• Y eine Gruppe Carbonyl oder eine Gruppe Methylen darstellt,
• R₆ eine Wasserstoffatom oder eine Seitenkette einer natürlichen Aminosäure ist, gegebenenfalls substituiert, und
• s eine ganze Zahl darstellt, die von 1 bis einschließlich 10 variieren kann, wobei, wenn s gleich 1 ist, R₆ eine Seitenkette einer natürlichen Aminosäure darstellt, gegebenenfalls substituiert, und wenn s höher als 1 ist, R₆ ein Wasserstoffatom darstellt,
**dadurch gekennzeichnet, daß** man eine Amidingruppe durch Einwirkung eines Thio- oder Oxo-Derivates von Harnstoff auf ein Lipopolyamin bildet oder daß man ein Polyaminoamidin auf ein Lipid durch peptidische Kopplung pfropft.

33. Methode zum Transfer von Nucleinsäuren in Zellen, **dadurch gekennzeichnet, daß** sie die folgenden Stufen umfaßt:
(1) das In-Kontakt-Bringen der Nucleinsäure mit einer Verbindung wie in den Ansprüchen 1 bis 20 definiert und gegebenenfalls mit einem oder mehreren Zusatzstoff(en) und/oder Ziel-Element(en), um einen Komplex zu bilden, und
(2) das In-Kontakt-Bringen der Zellen mit dem in (1) gebildeten Komplex.

## Claims

1. Lipopolyamine in D, L or DL form, as well as its salts, of general formula (I) : 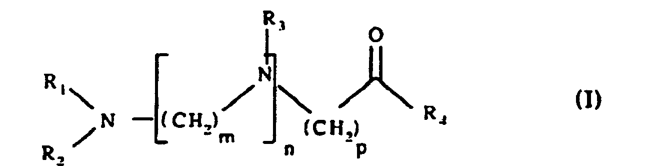 in which:
- R₁, R₂ and R₃ represent, independently of each other, a hydrogen atom or a group -(CH₂)_{q}-NRR' with
• q being an integer from 1 to 6 inclusive, the values of q being independent of each other between the different groups R₁, R₂ and R₃, and
• R and R' represent, independently of each other, a hydrogen atom or a group of formula (II): 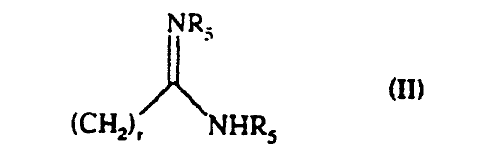
in which r is an integer which may vary from 0 to 6 inclusive, and the R₅ groups represent, independently of each other, a hydrogen atom or an optionally halogenated aliphatic or aromatic acyl, carbamate or alkyl substituent,
it being understood that at least one of the groups R₁, R₂ and R₃ contains at least one group of formula (II);
- m and n represent, independently of each other, an integer which may vary from 1 to 6 inclusive with, when n is greater than 1, m being capable of having different values and R₃ different meanings within the general formula (I),
- p represents an integer which may vary between 1 and 6 inclusive, and
- R₄ represents a group of general formula (III): 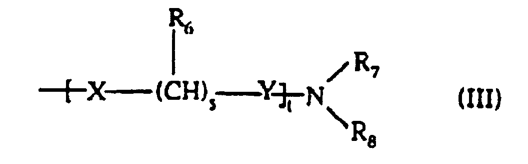
in which:
• R₇ and R₈ represent, independently of each other, a hydrogen atom or an optionally halogenated, linear or branched saturated or unsaturated aliphatic radical containing 5 to 22 carbon atoms, or a steroid derivative, or a (CH₂)ᵤ-NH-R₉ group in which u is an integer between 2 and 6 inclusive and R₉ is an acyl radical, at least one of the groups R₇ and R₈ being different from hydrogen,
• t is an integer chosen from 0 to 10 inclusive with R₆, X, Y and s being capable of having different meanings within the different units [X-(CHR₆)ₛ-Y] when t is an integer greater than 1,
• X represents an oxygen or sulphur atom or an amine or alkylamino group, the alkyl substituent being linear or branched and containing 1 to 8 carbon atoms,
• Y represents a carbonyl group or a methylene group,
• R₆ represents a hydrogen atom or a natural amino acid side chain, substituted where appropriate and
• s represents an integer varying from 1 to 10 inclusive with, when s is equal to 1, R₆ representing a natural amino acid side chain, substituted where appropriate, and when s is greater than 1, R₆ representing a hydrogen atom.

2. Compounds according to Claim 1, **characterized in that**, in formula (II), one of the groups R₅ represents a hydrogen atom and the other an aliphatic residue containing 1 to 10 carbon atoms or an aromatic residue.

3. Compounds according to Claim 1, **characterized in that**, in formula (II), one of the R₅ groups represents a hydrogen atom and the other benzyl or one of its derivatives.

4. Compounds according to Claim 1, **characterized in that**, in formula (II), the two R₅ groups represent hydrogen atoms.

5. Compounds according to one of the preceding claims, **characterized in that**, when R₁, R₂ and/or R₃ are different from hydrogen and comprise the formula (II), q takes the value 2 or 3 and r is equal to 0.

6. Compounds according to one of the preceding claims, **characterized in that**, in the general formula (I), m is chosen from 2, 3 and 4.

7. Compounds according to one of the preceding claims, **characterized in that** at least one of the groups R₇ and R₈ is represented by a steroid derivative chosen from cholesterol, cholestanol, 3-α-5-cyclo-5-α-cholestan-6-β-ol, cholic acid, cholesteryl formate, cholestanyl formate, 3α,5-cyclo-5α-cholestan-6β-yl formate, cholesterylamine, 6-(1,5-dimethylhexyl)-3a,5a-dimethylhexadecahydrocyclopenta[a]cyclopropa[2,3]cyclopenta[1,2-f]naphthalen-10-ylamine, or cholestanylamine.

8. Compounds according to one of Claims 1 to 6, **characterized in that** at least one of the groups R₇ and R₈ is represented by a group (CH2)ᵤ-NH-R₉ in which u is an integer between 2 and 6 inclusive and R₉ is an acyl radical.

9. Compounds according to Claim 8, **characterized in that** the R₉ group is cholesteryl formate, arachidonyl or cholic acid.

10. Compounds according to Claim 1, **characterized in that** the two groups R₇ and R₈ represent, independently of each other, an optionally halogenated, linear or branched, saturated or unsaturated aliphatic radical containing 5 to 22 carbon atoms, or a steroid derivative, or a (CH₂)ᵤ-NH-R₉ group in which u is an integer between 2 and 6 inclusive and R₉ is an acyl radical.

11. Compounds according to Claim 10, **characterized in that** the two groups R₇ and R₈ represent an aliphatic chain containing 5 to 22 carbon atoms, and preferably between 12 and 22 carbon atoms.

12. Compounds according to Claim 1, **characterized in that** R₁ comprises a group of formula (II) and R₂ and R₃ are hydrogen atoms.

13. Compounds according to Claim 1, **characterized in that** R₁ and R₂ each comprise a group of formula (II) and R₃ is a hydrogen atom.

14. Compounds according to Claim 1, **characterized in that** R₁ and R₃ each comprise a group of formula (II) and R₂ is a hydrogen atom.

15. Compounds according to Claim 1, **characterized in that** R₁, R₂ and R₃ each comprise a group of formula (II).

16. Compounds according to one of the preceding claims, **characterized in that** it combines with an extra- or intracellular targeting component.

17. Compounds according to Claim 16, **characterized in that** it incorporates the targeting component at the level of the R₆ amino acid side chain.

18. Compounds according to one of the preceding claims, **characterized in that** it incorporates a marker of the type including biotin, rhodamine, folate, or a linear or cyclic peptide or pseudopeptide sequence comprising the Arg-Gly-Asp epitope at the level of the R₆ amino acid side chain.

19. Compounds according to Claim 1, **characterized in that** they are chosen from the compounds of the following formulae in which R₄ and R₅ have the meanings given in Claim 1:

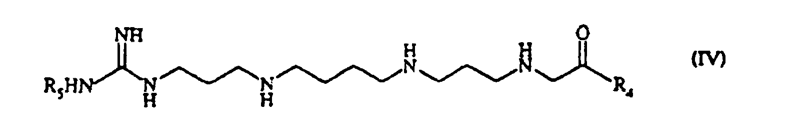

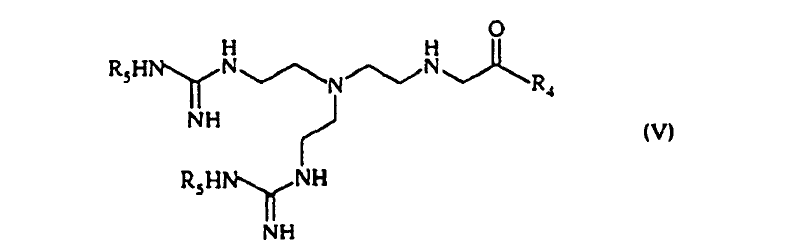

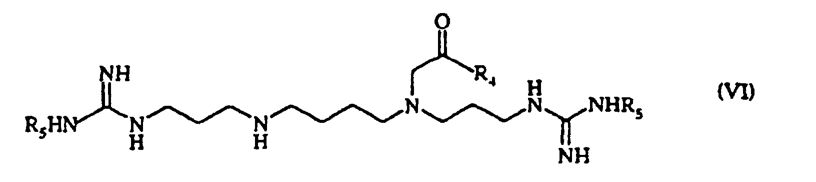

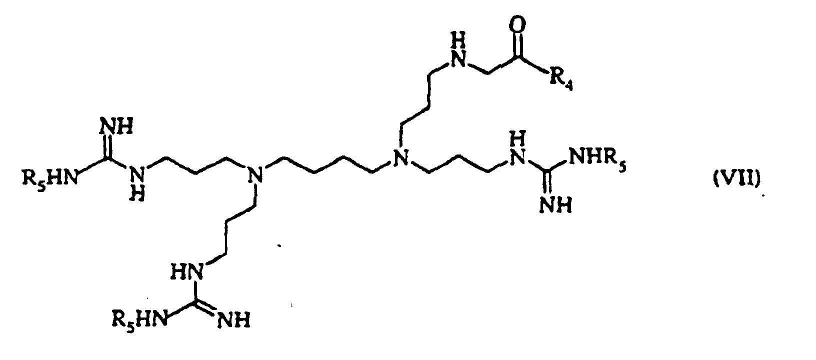

20. Compounds according to Claim 1, **characterized in that** they are chosen from:

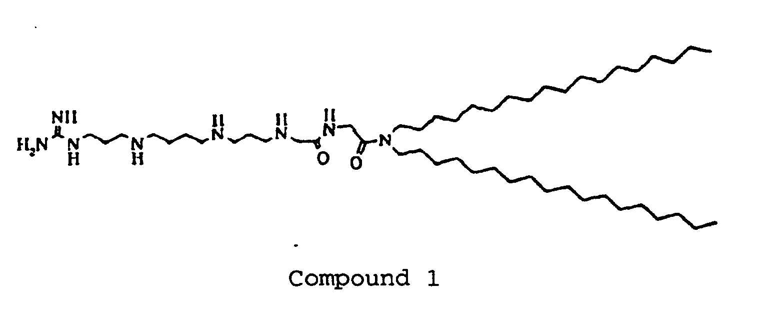

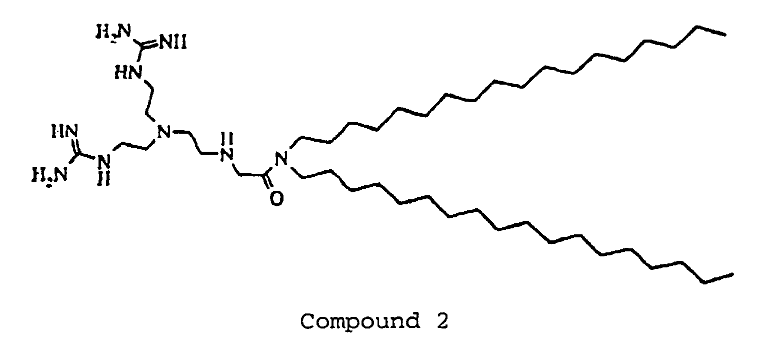

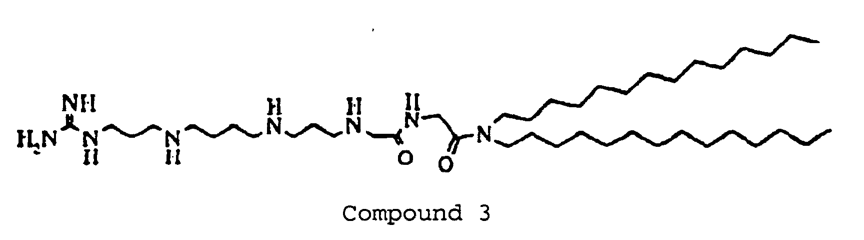

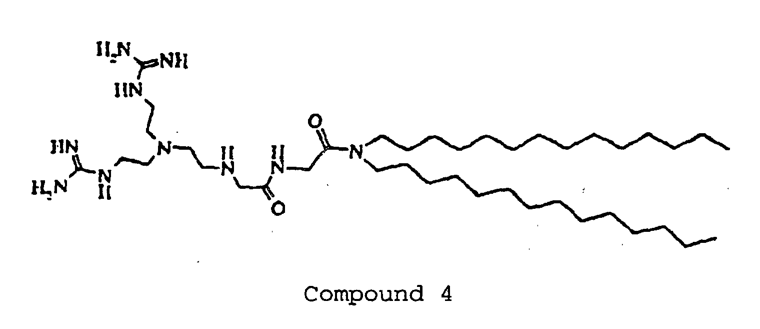

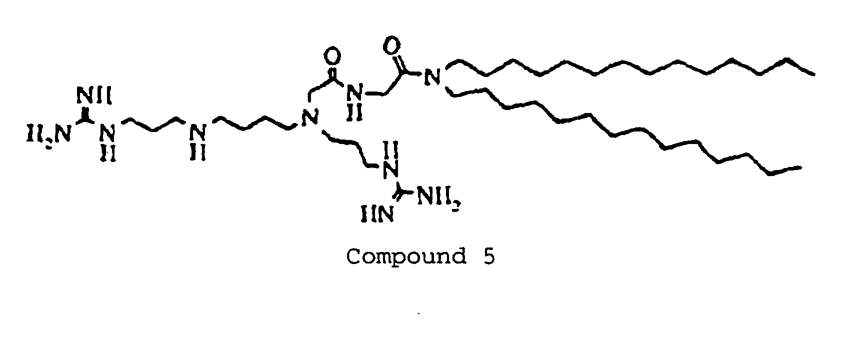

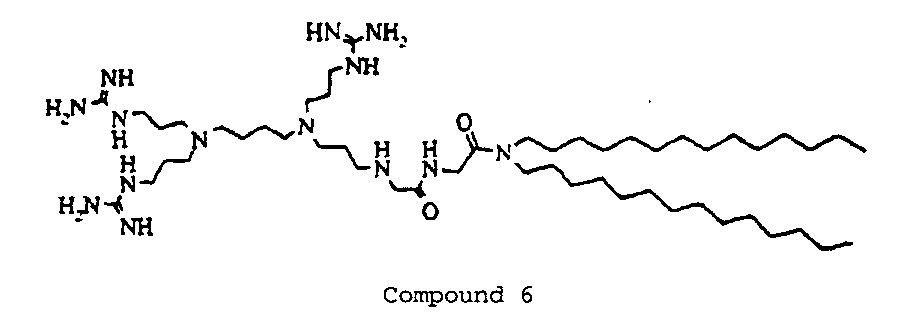

21. Composition comprising a compound according to one of the preceding claims and a nucleic acid.

22. Composition according to Claim 21, **characterized in that** it comprises, in addition, one or more adjuvants adjuvants.

23. Composition according to Claim 22, **characterized in that** the adjuvant is one or more neutral lipids.

24. Composition according to Claims 22 and 23, **characterized in that** the neutral lipids are lipids containing two fatty chains.

25. Composition according to Claims 22 to 24, **characterized in that** the neutral lipids are natural or synthetic lipids which are zwitterionic or lacking ionic charge under physiological conditions, chosen for example from dioleoylphosphatidylethanolamine (DOPE), oleoylpalmitoylphosphatidylethanolamine (POPE), di-stearoyl-, -palmitoyl- and -myristoyl-phosphatidylethanolamines as well as their derivatives which are N-methylated 1 to 3 times; phosphatidyl-glycerols, diacylglycerols, glycosyldiacylglycerols, cerebrosides (such as in particular galacto-cerebrosides), sphingolipids (such as for example sphingomyelins), or asialogangliosides (such as in particular asialoGM1 and GM2).

26. Composition according to Claim 22, **characterized in that** the adjuvant is a compound involved directly or otherwise at the level of the condensation of the nucleic acid.

27. Composition according to Claims 22 to 24, **characterized in that** the said adjuvant is derived as a whole or in part from a protamine, a histone, or a nucleolin and/or from one of their derivatives, or consists, as a whole or in part, of peptide motifs (KTPKKAKKP) and/or (ATPAKKAA), it being possible for the number of motifs to vary between 2 and 10 and to be repeated continuously or otherwise.

28. Composition according to Claim 21, **characterized in that** it comprises, in addition, a targeting component.

29. Composition according to Claims 21 to 28, **characterized in that** it comprises a vehicle which is pharmaceutically acceptable for an injectable formulation.

30. Composition according to Claims 21 to 28, **characterized in that** it comprises a vehicle which is pharmaceutically acceptable for application to the skin and/or the mucous membranes.

31. Use of a compound according to one of Claims 1 to 20 for transferring nucleic acids into cells.

32. Method of preparing a compound of general formula (I): 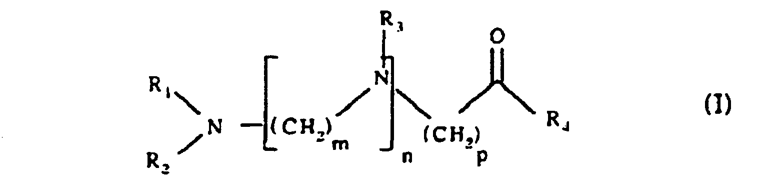 in which:
- R₁, R₂ and R₃ represent, independently of each other, a hydrogen atom or a group -(CH₂)_{q}-NRR' with
• q being an integer from 1 to 6 inclusive, the values of q being independent of each other between the different groups R₁, R₂ and R₃, and
• R and R' represent, independently of each other, a hydrogen atom or a group of formula (II): 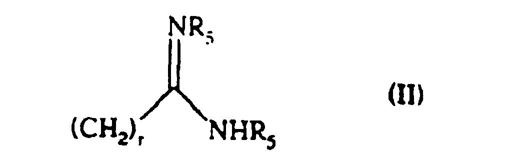
in which r is an integer which may vary from 0 to 6 inclusive, and the R₅ groups represent, independently of each other, a hydrogen atom or an optionally halogenated aliphatic or aromatic acyl, carbamate or alkyl substituent,
it being understood that at least one of the groups R₁, R₂ and R₃ contains at least one group of formula (II);
- m and n represent, independently of each other, an integer which may vary from 1 to 6 inclusive with, when n is greater than 1, m being capable of having different values and R₃ different meanings within the general formula (I),
- p represents an integer which may vary between 1 and 6 inclusive, and
- R₄ represents a group of general formula (III): 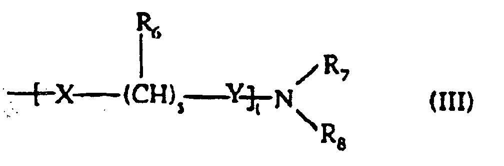
in which:
• R₇ and R₈ represent, independently of each other, a hydrogen atom or an optionally halogenated, linear or branched saturated or unsaturated aliphatic radical containing 5 to 22 carbon atoms, or a steroid derivative, or a (CH₂)ᵤ-NH-R₉ group in which u is an integer between 2 and 6 inclusive and R₉ is an acyl radical, at least one of the groups R₇ and R₈ being different from hydrogen,
• t is an integer chosen from 0 to 10 inclusive with R₆, X, Y and s being capable of having different meanings within the different units [X-(CHR₆)ₛ-Y] when t is an integer greater than 1,
• X represents an oxygen or sulphur atom or an amine or alkylamino group, the alkyl substituent being linear or branched and containing 1 to 8 carbon atoms,
• Y represents a carbonyl group or a methylene group,
• R₆ represents a hydrogen atom or a natural amino acid side chain, substituted where appropriate and
• s represents an integer varying from 1 to 10 inclusive with, when s is equal to 1, R₆ representing a natural amino acid side chain, substituted where appropriate, and when s is greater than 1, R₆ representing a hydrogen atom,
**characterized in that** an amidine group is formed by reacting a thio or oxo derivative of urea with a lipopolyamine or **in that** a polyaminoamidine is grafted on a lipid by peptide coupling.

33. Method of transferring nucleic acids into cells, **characterized in that** it comprises the following steps:
(1) bringing the nucleic acid into contact with a compound as defined in Claims 1 to 20 and, if appropriate, with one or more adjuvants and/or targeting component to form a complex and
(2) bringing the cells into contact with the complex formed in (1).
